# EUROPEAN PATENT SPECIFICATION

(11) **EP 4 021 416 B1**
(45) Date of publication and mention of the grant of the patent: **07.10.2026**
(21) Application number: 20768820.1
(22) Date of filing: 28.08.2020
(51) Int. Cl.: A61K 9/14, A61K 9/16, A61K 9/20, A61K 31/496, A61K 31/427, A61P 31/10

(54) **DRUG-POLYMER AMORPHOUS SOLID DISPERSIONS USING LINEAR POLY(ACRYLIC ACID) POLYMERS**
AMORPHE FESTE ARZNEIMITTEL-POLYMER-DISPERSIONEN UNTER VERWENDUNG VON LINEAREN POLY(ACRYLSÄURE)-POLYMEREN
DISPERSIONS SOLIDES AMORPHES DE POLYMÈRE DE MÉDICAMENT UTILISANT DES POLYMÈRES DE POLY(ACIDE ACRYLIQUE) LINÉAIRES

(30) Priority: 28.08.2019 US 201962892679 P
(43) Date of publication of application: 06.07.2022
(73) Proprietor: Lubrizol Advanced Materials, Inc., Cleveland, OH 44141-3247 (US)
(72) Inventor: DRAGANOIU, Elena S., Vaughan, ON L4L 8K8 (CA); MORGAN, Barbara J., Cleveland, OH 44141-3247 (US); WEAVER, David W., Avon Lake, OH 44012-2313 (US); SHAH, Shreya P., Bethlehem, PA 18017 (US); WILSON, Todd J., Bethlehem, PA 18017 (US); GRIECO, Michael, Bethlehem, PA 18017 (US); RANDALL, Cynthia S., Bethlehem, PA 18017 (US); ZUBRIS, Kimberly A., Bethlehem, PA 18017 (US); MITCHNICK, Mark A., East Hampton, NY 11937 (US); MIINEA, Liliana A., Cleveland, OH 44141-3247 (US)
(74) Representative: dompatent
(86) International application number: PCT/US2020/048429
(87) International publication number: WO 2021/041824

(56) References cited:
- WO-A1-2005/117834
- WO-A1-97/44014
- US-A1- 2015 011 525
- US-A1- 2016 256 433
- US-A1- 2017 014 346
- TIAN XIE ET AL: "Improved Release of Celecoxib from High Drug Loading Amorphous Solid Dispersions Formulated with Polyacrylic Acid and Cellulose Derivatives", MOLECULAR PHARMACEUTICS, vol. 13, no. 3, 2 February 2016 (2016-02-02), US, pages 873 - 884, XP055745298, ISSN: 1543-8384, DOI: 10.1021/acs.molpharmaceut.5b00798

## Description

### BACKGROUND

Aspects of the exemplary embodiment relate to an amorphous solid dispersion of an active pharmaceutical ingredient and to a method of forming an amorphous solid dispersion of an active pharmaceutical ingredient.

A number of drugs have been developed that have low aqueous solubility and thus poor oral bioavailability. These are generally classified in the Biopharmaceutics Classification System (BCS) in class II (high permeability, low solubility) and class IV (low permeability, low solubility). The pharmaceutical industry is faced with the challenge of formulating such drugs in a finished pharmaceutical product.

Increasing the solubility of a drug candidate while retaining its efficacy has proved to be a complex problem to solve. Approaches which have been proposed to address the problem include a) formation of salts for ionizable drugs; b) solutions in solvents, co-solvents and lipids; c) micelle systems; d) particle size reduction; e) complexation; f) prodrugs; g) amorphous solid dispersions. However, these techniques all have limitations, such as low aqueous solubility and thus poor oral bioavailability of a physically stable drug form.

WO2005117834A1, entitled SOLID DISPERSIONS OF A BASIC DRUG COMPOUND AND A POLYMER CONTAINING ACIDIC GROUPS, describes a solid dispersion including at least one basic drug compound and at least one pharmaceutically acceptable water-soluble polymer containing acidic groups, such as polyacrylic acid or polymethacrylic acid. The solid dispersion is formed by blending the components, extruding the blend at a temperature in the range of 20 - 300 °C, grinding the extrudate, and optionally sieving the particles.

U.S. Pub. No. 20100280047A1 published November 4, 2010, entitled SALTS OF ACTIVE INGREDIENTS WITH POLYMERIC COUNTER-IONS, by Kolter; et al., describes polymeric water-soluble salts of medicaments that are sparingly soluble in water, including a polymer with anion character that is soluble in water at pH values of 2-13, such as polyacrylic acid, and a sparingly soluble medicament with cation character. The salts are formed by dissolving the polymer and medicament in a solvent and precipitating the salt from the solution.

U.S. Pub. No. 20150011525A1, published January 8, 2015, entitled SOLID DISPERSION OF POORLY SOLUBLE COMPOUNDS COMPRISING CROSPOVIDONE AND AT LEAST ONE WATER-SOLUBLE POLYMER, by Bi, et al., describes a stable ternary solid dispersion composition including 1-50% wt. of one or more poorly soluble active pharmaceutical ingredient belonging to BCS class II and/or IV; 11-50% wt. of at least one water-soluble polymer, such as a homo- or co-polymer of acrylic acid or methacrylic acid; and 20-99% wt. of crosslinked polyvinylpyrrolidone (crospovidone, a water-insoluble polymer). The method of forming the solid dispersion includes preparing a homogenous aqueous and/or organic solution of the polymer and active pharmaceutical ingredient; suspending crosslinked polyvinylpyrrolidone in the resultant solution to yield a suspension or dispersion; and spray-drying the resultant suspension or dispersion to yield a dry powder form of a solid dispersion composition.

WO2014135545, entitled SOLID DISPERSION COMPRISING AMORPHOUS LORCASERIN HYDROCHLORIDE, describes an amorphous solid dispersion including lorcaserin hydrochloride and a pharmaceutically acceptable water soluble polymer, such as a polyacrylic acid. The method of forming the solid dispersion includes forming a solution of lorcaserin in a suitable solvent; adding a solution providing hydrogen chloride; optionally, concentrating the obtained composition; adding a water-soluble polymer and a suitable solvent; and optionally spray-drying the composition.

U.S. Pub. No. 20170014346A1, published January 19, 2017, entitled SPRAY DRYING PROCESS FOR PRODUCTION OF POWDERS WITH ENHANCED PROPERTIES, by Santos, et al., describes a spray drying method for production of amorphous solid dispersions which includes providing a feed mixture including an active pharmaceutical ingredient, one or more excipients such as polyacrylate or polymethacrylate, and a solvent; feeding the feed mixture to a spray drying apparatus; atomizing the feed mixture into droplets using an atomization nozzle; drying the droplets with a drying gas to produce particles; feeding a secondary gas stream at a separate location of the spray drying apparatus; and recovering the particles from the spray dryer chamber.

U.S. Pub. No. 20160256433A1, published September 8, 2016, entitled FORMULATIONS CONTAINING AMORPHOUS DAPAGLIFLOZIN, by Staric, et al., describes an amorphous solid dispersion including dapagliflozin and a polymer, such as polyacrylic acid The method of forming the amorphous solid dispersion includes preparing a solution of dapagliflozin and polymer in a suitable solvent; spraying or dispersing the solution onto carrier particles to form granules; evaporating the solvent; and blending the obtained composition with one or more pharmaceutically acceptable excipients.

WO 97/44014 A1, entitled ANTIFUNGAL COMPOSITIONS WITH INCREASED BIOAVAILABILITY, describes compositions containing itraconazole and a water-soluble polymer, formed by melt-extrusion.

Tian Xie, et al., "Improved Release of Celecoxib from High Drug Loading Amorphous Solid Dispersions Formulaled with Polyacrylic Acid and Cellulose Derivatives," MOLECULAR PHARMACEUTICS, vol. 13, no. 3, pp. 873-884 (2016), describes amorphous solid dispersions using a binary combination of polyacrylic acid and hydroxypropyl methyl cellulose as a stabilizer.

Although several solid dispersions including drugs and polymers are known in the art, there remains a need for improving the drug loading levels in the amorphous solid dispersions, while maintaining satisfactory storage stability of amorphous solid dispersions. A formulation method is described that can be applied to a broad range of active pharmaceutical ingredients and drug candidates that belong to BCS class II and IV, with flexibility (broad range) of drug loading levels and acceptable storage stability

### BRIEF DESCRIPTION

In accordance with one aspect of the exemplary embodiment, an **amorphous solid dispersion includes at least 10 wt.% of a linear poly(acrylic acid); and an active pharmaceutical ingredient, wherein: the linear poly(acrylic acid) has a Brookfield viscosity at 25°C of at least 100 mPa·s (at least 100 cP), the linear poly(acrylic acid) and the active pharmaceutical ingredient together comprise at least 80 wt.% of the amorphous solid dispersion, and the linear poly(acrylic acid) has a weight average molecular weight of at least 200,000 g/mol (at least 200,000 Da).**
**The dynamic viscosity is expressed in the description as centipoise (cP). One cP is equal to one millipascal second (mPa·s).**

In various aspects of the amorphous solid dispersion:
a) a ratio by weight of active pharmaceutical ingredient: poly(acrylic acid) in the amorphous solid dispersion is at least 1:10, or at least 1:6, or at least 1:3, or at least 1:1.5, or at least 1:1, or at least 2:1, or at least 3:1, or at least 4:1, or up to 6:1, or up to 5:1, or up to 4.5:1; e.g., from 1:10 to 5:1, or 1:6 to 4.5:1;
b) the Brookfield viscosity at 25°C of the linear poly(acrylic acid) is at least 200 cP, or at least 250 cP, or at least 300 cP, or at least 400 cP, and/or the linear poly(acrylic acid) has a Brookfield viscosity at 25°C of no more 3000 cP, or no more than 2,500 cP, or no more than 2200 cP, or no more than 2100 cP; e.g., from 200 cP to 3000 cP, or 250 cP to 2,500 cP;
c) the amorphous solid dispersion includes at least 10 wt.% linear poly(acrylic acid), or at least 15 wt.% linear poly(acrylic acid), or at least 20 wt.% linear poly(acrylic acid), or at least 25 wt.% linear poly(acrylic acid) and/or the amorphous solid dispersion comprises no more than 95 wt.% linear poly(acrylic acid), or no more than 80 wt.% linear poly(acrylic acid), or no more than 60 wt.% linear poly(acrylic acid), or no more than 50 wt.% linear poly(acrylic acid), or no more than 40 wt.% linear poly(acrylic acid), or no more than 30 wt.% linear poly(acrylic acid); e.g., from 10 wt.% to 95 wt.% linear poly(acrylic acid), or from 15 wt.% to 80 wt.% linear poly(acrylic acid);
d) the linear poly(acrylic acid) and the active agent together constitute at least 80 wt.%, or at least 90 wt.%, or at least 95 wt.% of the amorphous solid dispersion, or up to 100 wt.% of the amorphous solid dispersion;
e) the amorphous solid dispersion comprises no more than 10 wt. % water, or no more than 5 wt. % water, or no more than 1 wt. % water, or no water;
f) the active pharmaceutical ingredient is in BCS class II or BCS class IV;
g) a product includes the described amorphous solid dispersion, and optionally at least one excipient or adjuvant;
h) the product is in a form selected from granules, capsules, pellets, tablets, films, and implants;
i) a method of administering an active pharmaceutical ingredient to a person or non-human animal in need of treatment includes orally administering an amorphous solid dispersion as described or the product as described to the person or animal; and
   combinations of these aspects.

In another aspect of the exemplary embodiment, a method of forming an amorphous solid dispersion of an active pharmaceutical ingredient includes forming a liquid dispersion of a linear poly(acrylic acid), an active pharmaceutical ingredient, and a solvent system, the linear poly(acrylic acid) having a Brookfield viscosity at 25°C of at least 100 cP, and evaporating the solvent system from the liquid dispersion to form an amorphous solid dispersion.

In various aspects of the method:
a) a weight ratio of active pharmaceutical ingredient: linear poly(acrylic acid) in the liquid dispersion is at least 15:85, or at least 30:70, or at least 40:60, or at least 50:50, or at least 70:30; and/or a weight ratio of active pharmaceutical ingredient: linear poly(acrylic acid) in the liquid dispersion is no more than 90:10, or no more than 85:15; e.g., from 15:85 to 90:10, or from 30:70 to 85:15;
b) the linear poly(acrylic acid) has a Brookfield viscosity at 25°C, of at least 200 cP, or at least 250 cP, or at least 300 cP, or at least 400 cP; and/or the linear poly(acrylic acid) has a Brookfield viscosity of no more 3000 cP, or no more than 2,500 cP, or no more than 2200 cP, or no more than 2100 cP; e.g., from 200 cP to 3000 cP, or 250 cP to 2,500 cP;
c) the linear poly(acrylic acid) is one which has been formed in a solvent system which is substantially free of water;
d) the linear poly(acrylic acid) is one which has been formed in a solvent system selected from a) ethyl acetate and b) a mixture of ethyl acetate and cyclohexane;
e) the amorphous solid dispersion includes at least 10 wt.% linear poly(acrylic acid), or at least 15 wt.% linear poly(acrylic acid), or at least 20 wt.% linear poly(acrylic acid), or at least 25 wt.% linear poly(acrylic acid); and/or no more than 95 wt.% linear poly(acrylic acid), or no more than 80 wt.% linear poly(acrylic acid), or no more than 60 wt.% linear poly(acrylic acid), or no more than 50 wt.% linear poly(acrylic acid), or no more than 40 wt.% linear poly(acrylic acid), or no more than 30 wt.% linear poly(acrylic acid); e.g., from 10 wt.% to 95 wt.% linear poly(acrylic acid), or from 15 wt.% to 80 wt.% linear poly(acrylic acid), or from 10 wt.% to 60 wt.% linear poly(acrylic acid);
f) the linear poly(acrylic acid) and the active agent together constitute at least 80 wt.%, or at least 90 wt.%, or at least 95 wt.% of the amorphous solid dispersion; and/or up to 100 wt.% of the amorphous solid dispersion;
g) the amorphous solid dispersion includes no more than 10 wt. % water, or no more than 5 wt. % water, or no more than 1 wt. % water, or no water;
h) the forming of the dispersion of the linear poly(acrylic acid) and the active pharmaceutical ingredient includes dissolving the linear poly(acrylic acid), in powder form in the solvent system or in at least one of a plurality of solvents used in the solvent system;
i) the solvent system includes at least one of an organic polar protic solvent and a polar aprotic solvent;
j) the solvent system includes at least one organic polar protic solvent selected from C1-C6 alcohols, and mixtures thereof;
k) the solvent system includes at least one polar aprotic solvent selected from dichloromethane, C₃-C₈ ketones, C₃-C₈ ethers, and mixtures thereof;
l) the active pharmaceutical ingredient is in BCS class II or BCS class IV;
m) the evaporating of the solvent system from the liquid dispersion includes spray drying;
n) the method further includes preparing a product comprising the amorphous solid dispersion, the product being selected from granules, capsules, pellets, tablets, films, and implants;
o) an amorphous solid dispersion formed by the method described.
p) a product includes the amorphous solid dispersion and at least one excipient or adjuvant;
q) the product is in a form selected from granules, capsules, pellets, tablets, films, and implants; and
   combinations of these aspects.

In accordance with another aspect of the exemplary embodiment, a linear polyacrylic acid that stabilizes BCS class II and IV active pharmaceutical ingredients as amorphous solid dispersions, having a Brookfield viscosity of at least 100 cP, or at least 200 cP, or at least 250 cP, or at least 300 cP, or at least 400 cP, at 25°C, such as no more 3000 cP, or no more than 2,500 cP, or no more than 2200 cP, or no more than 2100 cP; e.g., from 200 cP to 3000 cP, or 250 cP to 2,500 cP.

In various aspects:
a) the linear polyacrylic acid is formed by a method in which a precursor monomer is polymerized in a solvent system which is substantially free of water;
b) the solvent system is selected from ethyl acetate and a mixture of ethyl acetate and cyclohexane;
c) the Brookfield viscosity is no more 3000 cP, or no more than 2,500 cP, or no more than 2200 cP, or no more than 2100 cP; and
   combinations of these aspects.

### BRIEF DESCRIPTION OF THE DRAWINGS

FIGURE 1 is a flow chart illustrating a method of forming a solid dispersion of an active pharmaceutical ingredient and in accordance with one aspect of the exemplary embodiment;

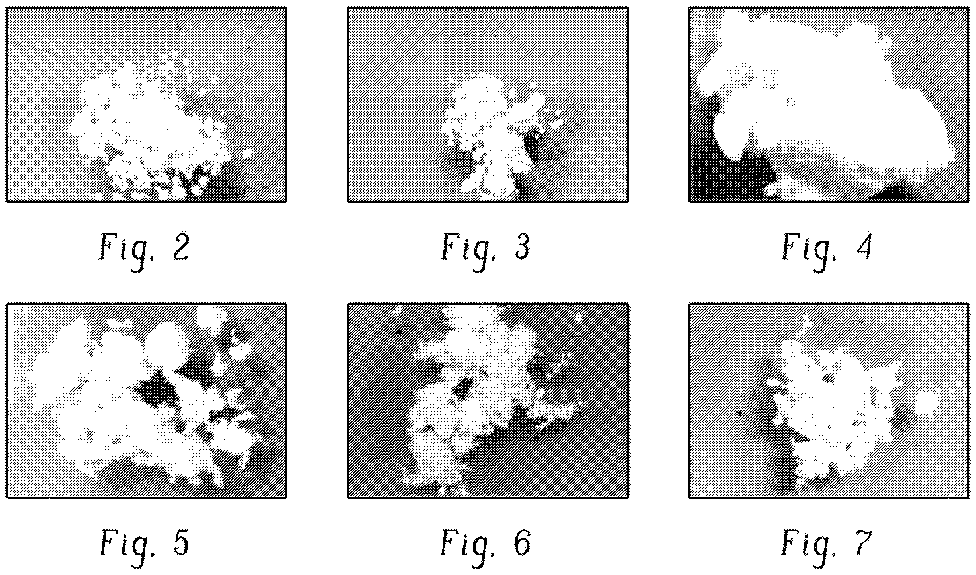

FIGURES 2-7 are photographs of spray-dried products at the same scale: FIG. 2 shows a product produced from 40% Itraconazole (ITZ) and 60% Soluplus^{®} polymer; FIG. 3 shows a product produced from 40% ITZ and 60% Affinisol^{®} polymer; FIG. 4 shows a product produced from 40% ITZ and 60% poly(acrylic acid) PAA (high molecular weight, formed in ethyl acetate cyclohexane cosolvent: HMW-CO); FIG. 5 shows a product produced from 40% ITZ and 60% PAA (medium molecular weight, formed in ethyl acetate cyclohexane cosolvent: MMW-CO); FIG. 6 shows a product produced from 40% ITZ and 60% PAA (low molecular weight, formed in ethyl acetate cyclohexane cosolvent LMW-CO); and FIG. 7 shows a product produced from 40% ITZ and 60% PAA (medium molecular weight, formed in ethyl acetate: MMW-EA);
FIGURES 8-10 show XRPD plots for products made from ITZ and PAA (high molecular weight, prepared in cosolvent: HMW-CO): FIG. 8 shows plots for ITZ alone, a physical mixture of 15% ITZ and 85% PAA, and a spray-dried solid dispersion of 15% ITZ and 85% PAA; FIG. 9 shows plots for ITZ alone, a physical mixture of 30% ITZ and 70% PAA, and a spray-dried solid dispersion of 30% ITZ and 70% PAA; and FIG. 10 shows plots ITZ alone, a physical mixture of 50% ITZ and 50% PAA, and a spray-dried solid dispersion of 50% ITZ and 50% PAA;
FIGURES 11-15 show Differential Scanning Calorimetry (DSC) plots: FIG. 11 shows a plot for a spray dried mixture of 70% ITZ and 30% PAA (medium molecular weight, formed in ethyl acetate: MMW-EA); FIG. 12 shows a plot for a spray dried mixture of 80% ITZ and 20% PAA (MMW-EA); FIG 13 shows a plot for a spray dried mixture of 90% ITZ and 10% PAA (MMW-EA); FIG. 14 shows a plot for a spray dried mixture of 80% ITZ and 20% Soluplus^{®} polymer; and FIG. 15 shows a plot for a spray dried mixture 80% ITZ and 20% Affinisol^{®} polymer;
FIGURE 16 shows chromatograms of the ITZ assay from 40% ITZ - 60% PAA (prepared in cosolvent, at high molecular weight, medium molecular weight, and low molecular weight: HMW-CO, MMW-CO and LMW-CO);
FIGURE 17 shows mean release of ITZ in 0.1N HCl under non-sink conditions from 15, 30 and 50% ITZ - PAA physical mixtures, 15, 30 and 50% ITZ - PAA spray dried amorphous solid dispersions (ASDs) (high molecular weight, prepared in co-solvent: HMW-CO), and ITZ neat and 100 % ITZ spray dried formulations;
FIGURE 18 shows mean ITV release under non-sink conditions in 0.1N HCl from 40% ITZ - 60% PAA spray dried ASDs (at high, medium, and low molecular weight, prepared in co-solvent: HMW-CO, MMW-CO, and LMW-CO), a 40% ITZ - 60% spray dried Soluplus^{®} ASD, and a spray dried 40% ITZ - 60% Affinisol^{®} ASD;
FIGURE 19 shows mean ITZ release under non-sink conditions in 0.1 N HCl from 40% ITZ - 60% PAA spray dried ASD (medium molecular weight, formed in ethyl acetate: MMW-EA),40% ITZ - 60% PAA spray dried ASD (low molecular weight, formed in cosolvent: LMW-CO), a 40% ITZ - 60% Soluplus^{®} ASD, and a 40% ITZ - 60% Affinisol^{®} ASD;
FIGURE 20 shows mean ITZ release (under non-sink conditions in 0.1N HCl from 80% ITZ - 20% PAA spray dried ASD (medium molecular weight PAA, formed in ethyl acetate: MMW-EA); 40% ITZ - 60% PAA spray dried ASD (medium molecular weight, formed in ethyl acetate: MMW-EA); 70% ITZ -30% PAA spray dried ASD medium molecular weight, formed in ethyl acetate: MMW-EA); and 40% ITZ - 60% Soluplus^{®} and 40% ITZ - 60% Affinisol^{®} spray dried ASDs;
FIGURE 21 shows ITZ release under non-sink conditions in HCl 0.1N from 80% ITZ - 20% PAA spray dried ASDs (low, medium, and high molecular weight PAA formed in ethyl acetate: LMW-EA, MMW-EA and HMW-EA);
FIGURE 22 shows mean release of ITZ in 0.1N HCl under non-sink conditions from 80%ITZ-20%PAA (MMW-EA) ASD as prepared (0 Months) and after 6 months storage in accelerated conditions (40°C/75% RH);
FIGURE 23 shows mean release of ITZ in 0.1N HCl under non-sink conditions from 40%ITZ-60% Soluplus^{®} ASD as prepared (0 Months) and after 6 months storage in accelerated conditions (40°C/75% RH);
FIGURE 24 shows mean release of ITZ in 0.1N HCl under non-sink conditions from 40%ITZ-60% Affinisol^{®} ASD as prepared (0 Months) and after 6 months storage in accelerated conditions (40°C/75% RH);
FIGURE 25 shows a Differential Scanning Calorimetry (DSC) plot for 80% ITZ - 20% Soluplus^{®} spray dried material stored at 40 °C/75%RH for 3 months; and
FIGURE 26 shows a Differential Scanning Calorimetry (DSC) plot for 80% ITZ - 20% Affinsiol^{®} spray dried material stored at 40 °C/75%RH for 3 months.

### DETAILED DESCRIPTION

Aspects of the exemplary embodiment relate to an amorphous solid dispersion of an active pharmaceutical ingredient, to a method for forming an amorphous solid dispersion of an active pharmaceutical ingredient, and to an amorphous solid dispersion formed by the method.

The exemplary amorphous solid dispersion includes a linear poly(acrylic acid) polymer and an active pharmaceutical ingredient. The polymer can stabilize drugs in amorphous form at up to 80% drug loading level, or more. The exemplary amorphous solid dispersion is formed by spray-drying, which is a reproducible and scalable pharmaceutical manufacturing process.

The exemplary method has several advantages over existing methods for preparing formulations of active pharmaceutical ingredients. These may include: improved aqueous solubility and thus improved oral bioavailability of a physically stable drug form (avoiding crystallization or phase separation of the amorphous drug); flexibility in drug loading levels (e.g., up to 80% or higher drug loading level) while maintaining stability of the amorphous solid dispersion; and manufacture of an amorphous solid dispersion by a reproducible and scalable process.

An "active pharmaceutical ingredient" (API) or "drug," as used herein, can be any substance or mixture of substances intended to be used in the manufacture of a drug product and that, when used in the production of a drug, becomes an active ingredient in the drug product. Such substances are intended to furnish pharmacological activity or other direct effect in the diagnosis, cure, mitigation, treatment or prevention of disease or to affect the structure and function of the body of an animal, such as a human.

The API may belong to BCS class II (high permeability, low solubility) or BCS class IV (low permeability, low solubility). A candidate API can be any substance or mixture of substances intended to be used in the manufacture of a drug product, which are being developed/undergoing testing for such use.

According to the US Food and Drug Administration, a drug in a solid dosage form is considered to be highly soluble when its highest clinical dose strength is soluble in 250 mL or less of aqueous media over a pH range of 1-6.8 at 37±1 °C, and it is considered to be highly permeable if the absorption of an orally administered dose in humans (denoted *fₐ*) is 85% or more, based on a mass balance determination (along with evidence showing stability of the drug in the GI tract) or in comparison to an intravenous reference dose. (See, "Waiver of In Vivo Bioavailability and Bioequivalence Studies for Immediate-Release Solid Oral Dosage Forms Based on a Biopharmaceutics Classification System: Guidance for Industry," U.S. Department of Health and Human Services, Food and Drug Administration, Center for Drug Evaluation and Research (CDER), at p. 3 (2017)), hereinafter "USFDA 2017".

In accordance with USFDA 2017, a low permeability API is one where the *fₐ* is less than 50%, determined in accordance with the method outlined in USFDA 2017. A low solubility API is considered herein to be one in which its highest clinical dose strength (as applicable in 2019) is not soluble in 250 mL or less of aqueous media over a pH range of 1-6.8 at 37±1 °C, determined in accordance with the method outlined in USFDA 2017. The aqueous solubility of the API may be less than 0.1 g/L or less than 0.05 g/L over a pH range of 1-6.8 at 37±1 °C.

The active pharmaceutical ingredient may be an analgesic, antiinflammatory agent, anthelmintic, anti-arrhythmic agent, antibacterial agent, antiviral agent, anticoagulant, antidepressant, antidiabetic agent, antiepileptic agent, antifungal agent, antigout agent, antihypertensive agent, antimalarial agent, antimigraine agent, antimuscarinic agent, antineoplastic agent, erectile dysfunction improvement agent, immune suppressant, antiprotozoal agent, antithyroid agent, anxiolytic agent, sedative, hypnotic, neuroleptic agent, β-blocker, cardiac inotropic agent, corticosteroid, diuretic, antiparkinsonian agent, gastro-intestinal agent, histamine receptor antagonist, keratolytic, lipid regulating agent, antianginal agent, Cox-2 inhibitor, leukotriene inhibitor, macrolide, muscle relaxant, opioid analgesic, protease inhibitor, sex hormone, muscle relaxant, antiosteoporosis agent, anti-obesity agent, cognition enhancer, anti-urinary incontinence agent, anti-benign prostate hypertrophy agent, antipyretic, muscular relaxant, anticonvulsant, antiemetic, anti-Alzheimer agent, or combination thereof.

Examples of BCS class II drugs include aceclofenac, acetaminophen, acyclovir, albendazole, amisulpride, aripiprazole, atorvastatin, azithromycin, benidipine, bicalutamide, candesartan cilexetil, carbamazepine, carvedilol, cefdinir, cefuroxime axetil, celecoxib, chloroquine, chlorpromazine, cilostazol, clarithromycin, clofazimine, clopidogrel, clozapine, cyclosporine, cyproterone, cisapride, danazol, dexamethasone, diazepam, diclofenac, diloxanide, ebastine, efavirenz, epalrestat, ethyl icosapentate, ezetimibe, fenofibrate, fluconazole, flurbiprofen, gefitinib, glibenclamide, glyburide, gliclazide, glimepiride, glipizide, griseofulvin, haloperidol, hydroxyzine, ibuprofen, imatinib, indinavir, irbesartan, isotretinoin, itraconazole, ketoconazole, ketoprofen, lamotrigine, levodopa, levothyroxine sodium, lopinavir, loratadine, lorazepam, manidipine, mebendazole, medroxyprogesterone, meloxicam, metaxalone, methylphenidate, metoclopramide, mosapride, mycophenolate mofetil, naproxen, nelfinavir, nevirapine, nicergoline, niclosamide, nifedipine, nisoldipine, olanzapine, orlistat, oxcarbazepine, phenytoin, pioglitazone, pranlukast, praziquantel, pyrantel, pyrimethamine, quetiapine, quinine, raloxifene, rebamipide, risperidone, ritonavir, rofecoxib, simvastatin, spironolactone, sulfasalazine, tacrolimus, tamoxifen, telmisartan, teprenone, ticlopidine, ursodeoxycholic acid, valproic acid, valsartan, verapamil, warfarin, and pharmaceutically acceptable salts thereof.

APIs that belong to BCS Class II are poorly soluble but are absorbed from the solution by the lining of the stomach and/or intestine.

Examples of BCS class IV drugs include acetazolamide, allopurinol, amphotericin B, atovaquone, bifonazole, bleomycin, buparvaquone, cefuroxime, chloroquine, chlorothiazide, cyclosporin, dapsone, diminazene stearate, diminazene oleate, doxycycline, furosemide, mefloquine, metronidazole, mitoxantrone, nalidixic acid, nimorazole, paclitaxel, paracetamol, pentamidine, primaquine, proteinase inhibitors, ritonavir, tinidazole, titanium metallocene dichloride, tobramycin, prostaglandins, saquinavir, vinblastine, vincristine, vindesine, vancomycin, vecuronium, and pharmaceutically acceptable salts thereof.

In the following examples, itraconazole (C₃₅H₃₈Cl₂N₈O₄) is used as an example BCS class II API and ritonavir (C₃₇H₄₈N₆O₅S₂) as an example BCS class IV API. Itraconazole (ITZ) is a broad spectrum anti-fungal compound with a melting point of 170°C. ITZ is a 1:1:1:1 racemic mixture of four diastereomers (two enantiomeric pairs), each possessing three chiral centers. The solubility of ITZ in water is about 1-4 ng/mL. ITZ exhibits very poor oral bioavailability owing to its insolubility in intestinal fluids. Ritonavir (RTV), sold under the trade name Norvir^{™}, is an antiretroviral medication used along with other medications to treat HIV/AIDS. This combination treatment is known as highly active antiretroviral therapy (HAART). Ritonavir exhibits low and variable oral bioavailability due to its poor aqueous solubility.

As used herein, "poly(acrylic acid)" (PAA) is a homopolymer of acrylic acid. By "homopolymer" it is meant that at least 90 mol % of the units in the polymer are derived from acrylic acid, or at least 95 mol %, or at least 98 mol %, or 100 mol % of the units in the polymer are derived from acrylic acid.

The exemplary PAA is linear, i.e., has substantially no crosslinking. By this, it is meant that crosslinking (or branching) occurs, on average (mean), at fewer than one in ten of the poly(acrylic acid) units in the longest chain of the polymer, or at fewer than one in twenty or one in fifty of the poly(acrylic acid) units in the longest chain of the polymer. The cross-link density can also be defined as the inverse of the molecular weight between cross-links (Mc), and may be no more than 0.0014, or no more than 0.0007.

The PAA polymer can thus be generally described by the formula: 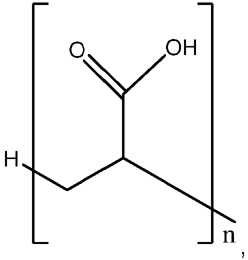 where n may be at least 1400, or at least 2000, or at least 3000, or at least 4000, or at least 5000, or at least 6000, or at least 8000, or at least 10,000, or at least 12,000, or at least 14,000, or up to 80,000.

The PAA used to form the amorphous solid dispersion (ASD) may have a high molecular weight, which can be expressed as a weight average molecular weight (M_{w}) or a number average molecular weight (Mₙ).

As used herein, the weight average molecular weight (M_{w}) is determined by size exclusion chromatography (SEC), as follows: a liquid sample is prepared of about 1.5 g/L (0.15%) polymer in 0.1M NaNO₃ at pH 10. The sample is filtered, prior to injection. 100 µL of the filtered sample is injected into the column (TOSOH Bioscience, 2x TSKgel PW_{XL} columns plus TSKgel Guard) using 0.1M NaNO₃ in deionized water at pH 10 as the mobile phase. The flow rate is 0.7 mL/min. A

Viscotek Triple Detector Array (TDA) (Malvern Panalytical) is used as the detector. This detector incorporates RI, Light Scattering, and Viscosity detectors. The instrument is calibrated with a single narrow MW standard of polyethylene oxide (PEO). A commercially-available sample of polyacrylic acid is used as a linear reference polymer. Mobile phase (and sample) enters the TDA and passes through the GPC/SEC chromatography columns. The columns are maintained at the same temperature as the detectors (40 °C). After eluting from the column, the dissolved polymer molecules, now separated by size, pass through the three detectors. Finally, the mobile phase passes through the viscometer before going to waste.

The RI detector gives information about the concentration of the components in the sample. The light scattering detectors respond to the intensity of light scattered by the sample which is related to molecular weight and also allows the Rg of large molecules to be calculated. The viscometer measures the changing solution viscosity to calculate the intrinsic viscosity of the sample (not used for viscosity determination herein).

The M_{w} of the PAA may be at least 200,000, or at least 250,000, or at least 300,000, or at least 400,000, or at least 500,000, or at least 600,000, or at least 800,000, or at least 1,000,000 Da. The M_{w} may be up to 10,000,000, or up to 5,000,000, or up to 3,000,000, or up to 2,000,000, or up to 1,500,000 Da. In one exemplary embodiment, the M_{w} ranges from 500,000 to 1,500,000.

As used herein, the number average molecular weight (Mₙ) is determined by the same method used for determining M_{w}. The Mₙ of the PAA may be at least 100,000 Da, or at least 120,000, or at least 140,000, or at least 150,000, or at least 160,000, or at least 180,000. The Mₙ may be up to 1,000,000, or up to 800,000, or up to 500,000 Da. In one exemplary embodiment, the Mₙ ranges from 150,000 to 500,000 Da.

As used herein, Brookfield viscosity is measured using a Brookfield viscometer model DV2TRV, 20 rpm, at 25 °C on an aqueous solution containing 4 wt. % of the PAA at pH 7.5. The spindles used with this model are RV01 - RV07 with the following viscosity range covered: RV-01, up to 500 cP; RV-02, up to 2000 cP; RV-03, up to 5000 cP; RV-04, up to 10,000 cP; RV-05, up to 20,000 cP: RV-06, up to 50,000 cP, and RV-07, up to 200,000 cP. The aqueous solution is formed by dissolving the PAA in water and adjusting the pH to 7.5 using an 18% aqueous solution of NaOH.

The PAA used to form the amorphous solid dispersion may have a Brookfield viscosity of at least 100 cP (cP=mPa·s), or at least 200 cP, or at least 250 cP, or at least 300 cP, or at least 400 cP, by this method. The viscosity may be up to 3,000 cp, or up to 2,500 cP, or up to 2200 cP or up to 2100 cP. In one exemplary embodiment, the Brookfield viscosity ranges from 200 to 2,200 cP.

Brookfield viscosity correlates well with molecular weight. Brookfield viscosity is proportional to molecular weight, as determined by the described methods. For example, a linear PAA polymer having a Brookfield viscosity of 200 cP has an Mₙ of 162,048 Da and an Mw of 545,692 Da; and a linear PAA polymer having a Brookfield viscosity of 2075 cP has an Mₙ of 527,772 Da and an M_{w} 1,071,000 Da.

Since Brookfield viscosity is more readily determined than molecular weight (Mₙ or M_{w}), it can be used as a molecular weight indicator.

PAA linear polymers, within the range of molecular weights stated above, may be described herein as low molecular weight (LMW), medium molecular weight (MMW), or high molecular weight (HMW). An example LMW polymer may have a Brookfield viscosity of 180-350 cP. An example MMW polymer may have a Brookfield viscosity of 400-1,000 cP. An example HMW polymer may have a Brookfield viscosity of 1,200-2,200 cP.

The exemplary linear PAA is in the form of a fine powder, which includes no more than 5 wt. % water, such as no more than 3 wt. % water, or no more than 2 wt. % water, or no more than 1 wt. % water. A water content of 2-3% water may occur due to the polymer hygroscopicity rather than a result of the synthesis. Water content is determined by the Loss on Drying method (LOD).

An "amorphous solid dispersion" (ASD), as used herein is, a dispersion of an API in a solid polymer matrix, which has substantially no crystalline character, as evidenced, for example, by commonly-used qualitative indicators of crystallinity, such as X-ray powder diffraction (XRPD), as described below, and Differential Scanning Calorimetry (DSC). In particular, crystalline character of a crystalline dispersion is evidenced by characteristic, well-defined peaks of the drug in the XRPD pattern and an obvious melting endotherm in the DSC thermogram. Absence of these indicators after spray-drying with linear PAA is consistent with an amorphous material. DSC may also be used to determine the glass transition temperature (Tg) of amorphous materials, which is not present in highly crystalline samples. An amorphous solid dispersion is also distinct from a physical blend of PAA and drug, in which the PAA and drug are simply combined by powder mixing.

The use of DSC and XRPD to characterize solid dispersions is well-known. For example, the application of XRPD and DSC to solid dispersions is described in Sóti, et al., "Comparison of Spray-drying, Electroblowing and Electrospinning for Preparation of Eudragit E and Itraconazole Solid Dispersions," Int. J. Pharm. 494:23, pp 1-27 (2015), and Wlodarski, et al., "Synergistic Effect of Polyvinyl Alcohol and Copovidone in Itraconazole Amorphous Solid Dispersions," Pharm. Res., 35:16, pp. 1 - 15 (2018).

Transmission or backscattering Raman spectroscopy may also be used. See, for example, Netchacovitch, et al., "Development of an analytical method for crystalline content determination in amorphous solid dispersions produced by hotmelt extrusion using transmission Raman spectroscopy: A feasibility study," Int. J. Pharm. 15, 530(1-2), pp. 249-255 (2017). As determined using transmission Raman spectroscopy, e.g., according to the method of Netchacovitch, et al., the percentage crystallinity of the amorphous sold dispersion may be less than 10%, or less than 5%, or less than 1%.

The exemplary amorphous solid dispersion includes, consists of, or consists essentially of poly(acrylic acid) and an API (or a mixture of APIs). By consists essentially of, it is meant that the polymer and API(s) together account for at least 90 wt. % (or at least 95 wt. %, or at least 98 wt. %) of the amorphous solid dispersion.

The amorphous solid dispersion may include at least 0.01 wt.% API, or at least 0.1 wt.% API, or at least 1 wt.% API, or at least 5 wt.% API, or at least 10 wt.% API, or at least 15 wt. % API, or at least 20 wt.% API, or at least 30 wt. % API or at least 40 wt.% API, or at least 50 wt.% API, or at least 60 wt.% API, or at least 70 wt.% API. The amorphous solid dispersion may include up to 90 wt.% API, or up to 85 wt.% API, or up to 80 wt.% API. As used herein the wt.% API (drug loading) is the weight of pure (undiluted) API(s) in the ASD. High loadings of API (e.g., 90 wt.% API, or above) may, in some cases, result in the solid dispersion being partially crystalline in character, which is undesirable for good solubility and absorption of the API. A rate of release of the drug from the solid dispersion is lower when the solid dispersion is partially crystalline.

The amorphous solid dispersion (ASD) includes at least 10 wt.% PAA polymer, and may include at least 15 wt.% PAA polymer, or at least 20 wt.% PAA polymer, or at least 25 wt.% PAA polymer. The ASD may include up to 99 wt.% PAA polymer, or up to 95 wt.% PAA polymer, or up to 80 wt.% PAA polymer, or up to 60 wt.% PAA polymer, or up to 50 wt.% PAA polymer, or up to 40 wt.% PAA polymer, or up to 30 wt.% PAA polymer.

A ratio, by weight, of active pharmaceutical ingredient: poly(acrylic acid) in the amorphous solid dispersion may be at least 1:10, or at least 1:6, or at least 1:3, or at least 1:1.5, or at least 1:1, or at least 2:1, or at least 3:1, or at least 4:1, or up to 6:1, or up to 5:1, or up to 4.5:1.

The exemplary amorphous solid dispersion includes no more than 5 wt. % water, or no more than 2 wt. % water, or no more than 1 wt. % water, such as no water.

In some embodiments, a formulation which includes the amorphous solid dispersion may further include one or more pharmaceutically acceptable excipients and/or adjuvants. The pharmaceutically acceptable excipient is an inert additive included in solid formulations to increase the bulk of the formulation comprising the ASD. The pharmaceutically acceptable adjuvant enhances the effectiveness of the API. The excipient(s) and/or adjuvant(s) may be added during or after the preparation of the spray-dried form of the amorphous solid dispersion.

In one embodiment, adjuvants and/or excipients may be present at up to a total of 99 wt. % of the formulation comprising the ASD, such as up to 20 wt. %, or up to 10 wt. %, or up to 5 wt. %. In one embodiment, adjuvants and/or excipients may be at least 0.01 wt. % of the formulation.

The exemplary amorphous solid dispersion may be formed from a liquid dispersion. A "liquid dispersion" is a system in which distributed particles of one material (here, at least the API and PAA) are dispersed in a continuous phase of another material (here, a solvent system). The two phases may be in the same or different states of matter. Liquid dispersions may be classified in a number of ways, including how large the particles are in relation to the particles of the continuous phase, whether or not precipitation occurs, and the presence of Brownian motion. In general, liquid dispersions of particles sufficiently large for sedimentation are referred to herein as suspensions, while those of smaller particles (which may be as little as on molecule in size) are referred to herein as colloidal mixtures or solutions.

The exemplary amorphous solid dispersion is formed by a solvent evaporation method, such as spray drying (SD). The amorphous solid dispersion may be in the form of an as-formed spray-dried powder, or may be further processed, e.g., to reduce the particle size and/or to form a product, e.g., in the form of granules, capsules, pellets, tablets, a film, a medical or dental implant, a dispersion of the ASD in a liquid medium, or an injectable product formulated for intravenous introduction to a human or non-human animal.

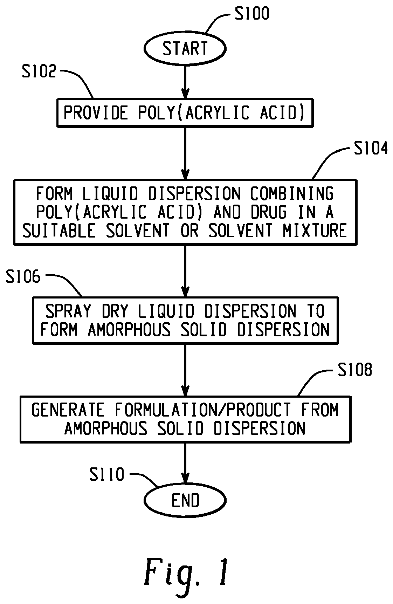

FIGURE 1 illustrates a method of forming the amorphous solid dispersion. The method begins at S100.

At S102, PAA is provided. This may include forming a PAA with a molecular weight and/or Brookfield viscosity as discussed above, or obtaining a preformed PAA. The PAA may be dissolved in a solvent or mixture of solvents in which the API is soluble.

At S104, the PAA and API are combined in a suitable organic solvent system, such as a single solvent or solvent mixture, to form a liquid dispersion, such as a solution, colloidal mixture, or suspension.

At S106, the liquid dispersion containing PAA polymer, API, and solvent is formed into ASD particles by spray drying or other solvent evaporation method.

At S108, a product comprising the thus-formed ASD may be prepared. This may include one or more of grinding, compacting into tablets, adding excipients and/or adjuvants, encapsulating the ASD in a shell, such as a material with a different solubility in water or stomach acid from the ASD, combinations thereof, and the like.

The method ends at S110.

### Preparation of PAA

The linear PAA polymer may be formed in solution, without addition of a cross-linking agent. The resulting linear PAA may be in the form of a powder.

Various methods exist for forming linear PAAs, which can be used to form a high molecular weight PAA. The PAA may be synthesized in a pharmaceutically-acceptable solvent system in which the starting material (e.g., acrylic acid monomer) is soluble. In one embodiment, the solvent is an organic solvent or mixture of organic solvents. Example organic solvents include ethyl acetate (EA), alone, or in combination with a co-solvent, such as a mixture of cyclohexane and ethyl acetate. A mixture of ethyl acetate and cyclohexane is referred to herein as CO. A ratio, by weight, of ethyl acetate : cyclohexane in the CO mixture may be from 30:70 to 100:0. The dispersion (e.g., solution) containing the monomer and solvent may be substantially free of water (non-aqueous). By this, it is meant that the solution includes no more than 10 wt. % water, or no more than 5 wt. %. water, or no more than 2 wt. % water, or 0 wt. % added water.

The PAA may be formed from acrylic acid monomer in the selected organic solvent in a free radical process, using an initiator, such as an organic peroxide. The reaction may be carried out at about room temperature, or above (e.g., 18-70°C). The acrylic acid may be partially pre-neutralized, prior to the polymerization, e.g., with sodium hydroxide. The degree of neutralization can be used to control the molecular weight of the PAA polymer. See, for example, Khanlari, et al., "Effect of pH on Poly(acrylic acid) Solution Polymerization," J. Macromolecular Science, Part A, 52:8, 587-592 (2015). In an organic solvent, such as ethyl acetate, the PAA forms as a precipitate, which can be used directly (after low temperature drying to remove most of the organic solvent) in the formation of the ASD, without the need for removing water from the PAA. For example, in the case of ethyl acetate and cyclohexane, drying may be performed at a temperature of below 90°C for less than 1 hour.

In other embodiments, the free radical reaction can also be carried out with the pure monomer (bulk polymerization), or by polymerization in an aqueous solution or an emulsion.

Poly(acrylic acid) may also be synthesized by anionic polymerization of t-butyl acrylate (e.g., with an organolithium reagent or other adduct initiator, and methyl alcohol) followed by acid hydrolysis of the tert-butyl group.

In another embodiment, the PAA is formed by a reversible addition-fragmentation transfer polymerization (RAFT) of acrylic acid, in the presence of a RAFT agent, such as trithiocarbonate. The molecular weight (Mₙ) of the resulting polymer can be controlled by selecting the ratio of [AA]:[RAFT agent]. See, for example, Ji, et al., "Efficient Synthesis of Poly(acrylic acid) in Aqueous Solution via a RAFT Process," J. Macromolecular Science, Part A, 47:5, 445-451 (2010). In the method of Ji, the chain transfer to solvent or polymer is suppressed during the polymerization process, thus high linear PAA with high molecular weight and low polydispersivity index (PDI) can be obtained. Moreover, using the generated PAA as a macro RAFT agent, the chain extension polymerization of PAA with fresh acrylic acid displays controlled behavior, demonstrated the ability of PAA to reinitiate sequential polymerization.

Poly(acrylic acid) with a volume average molecular weight (Mᵥ) of about 130,000, about 250,000, about 450,000, about 1,250,000, and about 3 million and about 4 million are available from Millipore Sigma or Sigma-Aldrich.

### Solvent Evaporation

Spray drying (SD) is a solvent evaporation process of producing a dry powder from a liquid by rapidly drying with a hot gas. While spray drying is used in the exemplary embodiment, other solvent evaporation processes which incorporate the evaporation of the solvent, e.g., a non-aqueous (organic) solvent are contemplated, e.g., under heat and/or vacuum, such as oven drying (e.g., film casting followed by oven drying which results in dry films of drug/polymer ASD); fluid bed drying (using a flow of air or other gas, resulting in dry powder); tumble drying (employing mechanical agitation, resulting in dry powder); electrospinning (resulting in nano- or micron- size fibers containing the ASD of drug/PAA); or electrospraying (resulting in a dry powder).

In the exemplary embodiment, where spray drying is used, the liquid supplied to the spray drier for spray drying includes PAA, at least one API, and a solvent, or mixture of solvents, in which the poly(acrylic acid) and API are soluble, specifically, more soluble than in water. Suitable solvents include polar protic organic solvents, such as C₁-C₆ alcohols, e.g., as ethanol, and polar (hydrophilic) aprotic solvents, such as dichloromethane (DCM), C₃-C₈ ketones, C₃-C₈ ethers, and other low-boiling organic solvents (e.g., a boiling point of less than 90 °C), and mixtures thereof. The solvent(s) evaporate from the liquid and thus are not present, or present only in minor amounts, in the amorphous solid dispersion. For example, the amorphous solid dispersion comprises less than 5 wt. % solvent, or less than 1 wt. % solvent.

For example, ethanol is a suitable solvent for RTV and a mixture of dichloromethane and ethanol is suitable for ITZ. A weight ratio of (Ethanol:DCM) in such a solvent system may be from 1:10 to 10:1, such as from 5:1 to 1:2, although any suitable solvent or solvent ratio which dissolves both the drug and polymer can be used.

A ratio of the combined weight of PAA and API to weight of solvent in the spray drying solution (or other dispersion) formed at S104 is not critical and may be, for example at least 0.015:1, or at least 0.02:1, and may be up to 0.2:1 or up to 0 1:1. A ratio of PAA to solvent, by weight, in the spray drying solution is not critical and may be, for example at least 0.01:1, or at least 0.02:1, and may be up to 0.19:1 or up to 0.09:1. A ratio of API to solvent, by weight, in the spray drying solution is not critical and may be, for example at least 0.008:1, or at least 0.015:1, and may be up to 0.09:1 or up to 0.07:1. A ratio of API to PAA in the spray drying solution may be selected based on the desired ratio in the ASD. For example, the ratio may be from 10:90 to 85:15 to achieve corresponding ratios of API to PAA in the ASD.

To form the spray drying solution, the PAA (e.g., in the form of a powder), and API may first be dissolved in respective solvent(s) (which may be the same or different) and the two liquids combined. In another embodiment, the neat API is added to a solution containing the PAA and solvent(s). In another embodiment, PAA in little or no solvent is added to a solution containing the API and a solvent. In some embodiments, the solution containing the PAA and API may incorporate one or more excipients and/or adjuvants or precursors therefor.

As an example, to form the spray drying solution, the PAA (e.g., in the form of a powder), and API may first be dissolved in respective solvent(s) (which may be the same or different) and the two liquids combined. Alternatively, the PAA may be dissolved in the solution of API in one solvent, and then the second solvent is added. The resulting mixture is pumped to a spray dryer to evaporate off the solvent(s) at temperature higher than the boiling point of the solvent(s) used, and the spray dried ASD is collected. For example, the inlet (maximum) temperature of the spray dryer may be at least 80°C, or at least 90°C in the case of ethanol (or ethanol:DCM mixtures). Ethanol boils at about 78°C, under atmospheric conditions. The inlet (maximum) temperature of the spray dryer may be up to 120°C, or up to 100°C for such solvents.

Residual organic solvent in the formed ASD may be less than 5 wt. %, or less than 2 wt. %, or less than 1 wt. %. The level of acceptable residual solvent(s) may depend on the type of solvent used (e.g., the acceptable amount for a class 1 or 2 solvent may be lower than for a (less toxic) class 3 solvent, as stipulated by pharmacopeial and/or regulatory guidance).

An active pharmaceutical ingredient may be administered orally to a person or animal in need of treatment in the form of a spray-dried amorphous solid dispersion formed by the exemplary method or in the form of a product formed from the spray-dried amorphous solid dispersion or administered by implanting an implant, such as a mesh or a tube of electrospun fibers, containing the ASD.

Without intending to limit the scope of the exemplary embodiment, the following examples demonstrate the drug loadings which can be achieved in an amorphous solid dispersion comprising poly(acrylic acid).

### EXAMPLES

### 1. Preparation of PAAs

Eight linear PAAs (PAAs 1-8) are synthesized in different solvents at a range of molecular weights (expressed as Brookfield viscosity, determined by the method described above). TABLE 1 shows the exemplary PAAs formed. EA denotes ethyl acetate and CO denotes a mixture of ethyl acetate and cyclohexane (e.g., EA: 30 wt. %, cyclohexane 70 wt. %). The poly(acrylic acid) products are defined as low (LMW), medium (MMW), or high (HMW) molecular weight, based on the Brookfield viscosity (determined as described above).

**TABLE 1: Linear poly(acrylic acid) polymers**

| Product | Synthesis Solvent | Brookfield viscosity (cP), 4% aq. pH 7.5 | MW Designation |
|---|---|---|---|
| PAA-1 | EA | 200 | LMW |
| PAA-2 | EA | 450 | MMW |
| PAA-3 | EA | 840 | MMW |
| PAA-4 | EA | 908 | MMW |
| PAA-5 | EA | 2075 | HMW |
| PAA-6 | CO | 278 | LMW |
| PAA-7 | CO | 750 | MMW |
| PAA-8 | CO | 1500 | HMW |

### 2. Model APIs

Itraconazole (ITZ) (1-(butan-2-yl)-4-{4-[4-(4-{[(2R, 4S)-2-(2, 4-dichlorophenyl)-2-(1H-1, 2, 4-triazol-1-ylmethyl)-1, 3-dioxolan-4-yl]methoxy}phenyl)piperazin-1-yl]phenyl}-4, 5-dihydro-1H-1, 2, 4-triazol-5-one), from Ra Chem Pharma Ltd. and SMS Pharma, and Ritonavir (RTV) (5-thiazolylmethyl ((alphaS)-alpha-((1S,3S)-1-hydroxy-3-((2S)-2-(3-((2-isopropyl-4-thiazolyl)methyl)-3-methylureido)-3-methylbutyramido)-4-phenylbutyl)phenethyl)carbamate), from LGM Pharma, polymorphic form II, were selected as low solubility model drugs. Properties of the two drugs are shown in TABLE 2.

**TABLE 2: Model APIs**

| Name | ltraconazole | Ritonavir |
|---|---|---|
| Property | synthetic triazole agent with antimycotic properties | antiretroviral |
| MW (g/moL) | 705.6 | 720.9 |
| pKa | 3.7 | 2.8 |
| Aqueous solubility | 1-4 µg/L | 0.00126 g/L |
| logP | 5.66 | 3.9 |
| Glass transition temperature, Tg (°C) | 52 | 45 |
| Tm (°C) | 166.8 | 125 |

The linear PAAs (in their respective synthesis solvents) are combined with the selected API and spray dried to obtain stable ASDs at various drug loadings (15 wt.%, 30 wt. %, 40 wt. %; 50 wt. % and 80 wt. %).

For comparison, spray dried mixtures of the drug alone and with other polymers: polyvinyl caprolactam-polyvinyl acetate-polyethylene glycol graft copolymer (PCL-PVAc-PEG) (Soluplus^{®}, BASF) and hydroxypropyl methyl cellulose (Affinisol^{®}, Dow) are also prepared (TABLE 3). These two polymers are commonly used to stabilize ASDs.

**TABLE 3: Comparative Polymers**

| Trade name | Soluplus^{®} | Affinisol^{®} 15 LV HME |
|---|---|---|
| MW (kDa) | 90 - 140 | 75 |
| Tg (°C) | 70 | 110 - 115 |
| Water solubility | Y | Y |
| Brookfield viscosity (cP) | < 50 | 15 |

### 3. Preparation of amorphous dispersions and other formulations

Spray dried formulations are prepared as follows.

For spray drying Ritonavir and PAA, the PAA (in the form of a powder) is dissolved in ethanol. The Ritonavir is also dissolved in ethanol. The two solutions are combined. The resulting solution is pumped to a spray-dryer (Büchi B-290) to evaporate off the solvent at temperature higher than the boiling point of the solvents used, and the spray dried dispersions are collected as powders.

For spray drying ITZ and PAA, ITZ is dissolved in DCM. PAA is dispersed in the resulting solution. Ethanol is added to the dispersion of PAA in ITZ-DCM to dissolve the PAA and to form a solution. The resulting solution is pumped to a spray-dryer (Büchi B-290) to evaporate off the solvents at temperature higher than the boiling point of the solvents used, and the spray dried dispersions are collected as powders.

For spray drying ITZ and Soluplus^{®} or ITZ and Affinisol^{®}: ITZ is dissolved in DCM. The polymer (Soluplus^{®} or Affinisol^{®}) is dissolved in DCM. The two solutions are combined. The resulting solution is pumped to a spray-dryer (Büchi B-290) to evaporate off the solvent at temperature higher than the boiling point of the solvents used, and the spray dried dispersions are collected as powders.

TABLE 4 shows the specific spray drying conditions for formulations F1-F15, prepared with ITZ. Dichloromethane (DCM) is used as the solvent for ITZ alone and a mixture of dichloromethane and ethanol, in various weight ratios (Ethanol:DCM), is used as a solvent for PAA:ITZ mixtures. TABLE 5 shows the spray drying conditions for formulations F16-F19, prepared with RTV, where ethanol is used as a solvent. TABLE 6 shows the spray drying conditions for formulations F20-F23, prepared with Affinisol^{®} and Soluplus^{®} polymers, where DCM is used as a solvent.

**TABLE 4: Spray Drying of ITZ and ITZ - PAA ASDs**

| **Process parameters** | **Formulations** | | | | |
|---|---|---|---|---|---|
| | **F1** | **F2** | **F3** | **F4** | **F5** |
| Drug, PAA | ITZ | 15% ITZ, 85% PAA-8 | 30% ITZ, 70% PAA-8 | 40% ITZ, 60% PAA-8 | 50% ITZ, 50% PAA-8 |
| Solvent system | DCM | Ethanol: DCM 3:1 | Ethanol: DCM 2:1 | Ethanol: DCM 2:1 | Ethanol: DCM 2:1 |
| Solvent (g) | 185 | 300:104* | 200:104 | 200:100 g | 200:104 |
| ITZ (g) | 10 | 3 | 3.6 | 4.8 | 7.5 |
| PAA (g) | -- | 17 | 8.4 | 7.2 | 7.5 |
| Wt. ratio of PAA: solvent | -- | 0.042 | 0.028 | 0.024 | 0.025 |
| Wt. ratio of ITZ: solvent | 0.054 | 0.007 | 0.012 | 0.016 | 0.025 |
| Wt. ratio of PAA+ITZ: solvent | 0.054 | 0.050 | 0.040 | 0.040 | 0.049 |
| Inlet Temp. (°C) | 60 | 95 | 95 | 95 | 95 |
| Outlet Temp. (°C) | 45 | 64 | 65 | 52 | 60 |
| Pump Rate (setting) | 5 | 3 | 3 | 6 | 3 |
| Aspirator rate | 100% | 100% | 100% | 100% | 100% |
| Yield | 5.18 g (51.8%) | 11.5 g (86.2%) | 11.10 g (92.5%) | 11.02 g (91.8%) | 11.7 g (78.0%) |

| | | | | | |
|---|---|---|---|---|---|
| * Sprayed only 2/3^{rd} of amount prepared so 13.34 g equivalent solution was sprayed therefore, yield as calculated based on that. | | | | | |

**TABLE 4, cont.**

| **Process parameters** | **Formulation** | | | | |
|---|---|---|---|---|---|
| | **F6** | **F7** | **F8** | **F9** | **F10** |
| Drug, PAA | 40% ITZ, 60% PAA-6 | 40% ITZ, 60% PAA-7 | 40% ITZ, 60% PAA-2 | 70% ITZ, 30% PAA-2 | 80% ITZ, 20% PAA-2 |
| Solvent system | Ethanol: DCM 2:1 | Ethanol: DCM 2:1 | Ethanol: DCM 1:1 | Ethanol: DCM 1.17:1 | Ethanol: DCM 1:1 |
| Solvent (g) | 200:100 | 200:100 | 200:201 | 234:200 | 200:200 |
| ITZ (g) | 4.8 | 4.8 | 4.8 | 8.4 | 9.6 |
| PAA (g) | 7.2 | 7.2 | 7.2 | 3.6 | 2.4 |
| Wt. ratio of PAA: solvent | 0.024 | 0.024 | 0.018 | 0.008 | 0.006 |
| Wt. ratio of ITZ: solvent | 0.016 | 0.016 | 0.012 | 0.019 | 0.024 |
| Wt. ratio of PAA+ITZ: solvent | 0.040 | 0.040 | 0.030 | 0.028 | 0.030 |
| Inlet Temp. (°C) | 95 | 95 | 95 | 95 | 95 |
| Outlet Temp. (°C) | 60 | 60 | 49 | 52 | 52 |
| Pump Rate (setting) | 6 | 6 | 6 | 6 | 6 |
| Aspirator rate | 100% | 100% | 100% | 90% | 100% |
| Yield | 11.10 g (92.5%) | 11 g (91.7%) | 10.28 g (85.7%) | 4.80 g (40%) | 3.50 g (29.2%) |

**TABLE 4, cont.**

| **Process parameters** | **Formulation** | | | | |
|---|---|---|---|---|---|
| | **F11** | **F12** | **F13** | **F14** | **F15** |
| Drug, PAA | 80% ITZ, 20% PAA-5 | 90% ITZ, 10% PAA-2 | 80%ITZ-20%PAA-1 | 80%ITZ-20%PAA-4 | 80%ITZ-20%PAA-3 |
| Solvent system | Ethanol: DCM 1:1 | Ethanol: DCM 1:1 | Ethanol: DCM 1.25:1 | Ethanol: DCM 1:1 | Ethanol: DCM 1:1 |
| Solvent (g) | 200:200 | 200:200 | 250:200 | 200:200 | 200:200 |
| ITZ (g) | 9.6 | 10.8 | 9.64 g | 9.60 g | 9.60 g |
| PAA (g) | 2.4 | 1.2 | 2.43 g | 2.40 g | 2.42 g |
| Wt. ratio of PAA: solvent | 0.006 | 0.003 | 0.005 | 0.006 | 0.006 |
| Wt. ratio of ITZ: solvent | 0.024 | 0.027 | 0.021 | 0.024 | 0.024 |
| Wt. ratio of PAA+ITZ: solvent | 0.030 | 0.030 | 0.027 | 0.030 | 0.030 |
| Inlet Temp. (°C) | 95 | 95 | 95 | 95 | 95 |
| Outlet Temp. (°C) | 49 | 54 | 61 | 61 | 58 |
| Pump Rate (setting) | 6 | 6 | 6 | 6 | 6 |
| Aspirator rate | 100% | 100% | 80% | 85% | 90% |
| Yield | 11.02 g (91.8%) | 5.70 g (47.5%) | 9.44 g (78.7%) | 7.82 g (65.2%) | 8.49 g (70.8%) |

**TABLE 5: Spray Drying of RTV and RTV - PAA ASDs**

| **Process parameters** | **Formulation** | | | |
|---|---|---|---|---|
| | **F16** | **F17** | **F18** | **F19** |
| Drug, PAA | RTV | 15% RTV, 85% PAA-8 | 30% RTV, 70% PAA-8 | 50% RTV, 50% PAA-8 |
| Solvent system | Ethanol | Ethanol | Ethanol | Ethanol |
| Solvent (g) | 312.87 | 390.45 | 195.92 | 195.91 |
| RTV (g) | 8.02 | 1.51 | 1.51 | 2.51 |
| PAA (g) | -- | 8.5 | 3.51 | 2.52 |
| Wt. ratio of PAA: solvent | -- | 0.022 | 0.018 | 0.013 |
| Wt. ratio of RTV: solvent | 0.026 | 0.004 | 0.008 | 0.013 |
| Wt. ratio of PAA+RTV: solvent | 0.026 | 0.026 | 0.26 | 0.026 |
| Inlet Temp. (°C) | 95 | 95 | 95 | 95 |
| Outlet Temp. (°C) | 65 | 60 | 64 | 65 |
| Pump Rate (setting) | 5 | 5 | 5 | 5 |
| Aspirator rate | 85% | 100% | 100% | 100% |
| Yield | 3.81 g (47.6%) | 7.33 g (73.3%) | 3.19 g (63.8%) | 3.27 g (65.4%) |

**TABLE 6: Spray Drying of ITZ- Soluplus^{®} and ITZ - Affinisol^{®}**

| **Process parameters** | **Formulation** | | | |
|---|---|---|---|---|
| | **F20** | **F21** | **F22** | **F23** |
| Drug, polymer | 40% ITZ, 60% Soluplus^{®} | 80% ITZ, 20% Soluplus^{®} | 40% ITZ, 60% Affinisol^{®} | 80% ITZ, 20% Affinisol^{®} |
| Solvent system | DCM | DCM | DCM | DCM |
| Solvent (mL) | 150 g | 300 g | 383.5 g | 250 g |
| ITZ (g) | 4.8 g | 9.6 g | 4.8 g | 9.6 g |
| Polymer (g) | 7.2 g | 2.4 g | 7.25 g | 2.4 g |
| Inlet Temp. (°C) | 58 | 58 | 58 | 58 |
| Outlet Temp.(°C) | 48 | 40 | 48 | 42 |
| Pump Rate (setting) | 8 | 9 | 8 | 9 |
| Aspirator rate | 90% | 90% | 90% | 90% |
| Yield | 4.5 g (37.5%) | 7 g (58.3%) | 7.2 g (60%) | 2 g (16.7%) |

Physical mixtures of linear PAAs and drug (without spray-drying) are also prepared, for comparison with the formulations made by spray drying. The selected drug and polymer are weighed separately and, using a geometric dilution method, are gently mixed together using a mortar and pestle. TABLE 7 lists these formulations.

**TABLE 7: Other Formulations**

| Formulation | Drug, Polymer | Drug: Polymer Ratio, by wt. |
|---|---|---|
| F24 | PAA-8 (polymer powder) | |
| F25 | ITZ (neat) | |
| F26 | ITZ, PAA-8 (physical mixture) | 15: 85 |
| F27 | ITZ, PAA-8 (physical mixture) | 30: 70 |
| F28 | ITZ, PAA-8 (physical mixture) | 50: 50 |
| F29 | RTV (neat) | |
| F30 | RTV, PAA-8 (physical mixture) | 15: 85 |
| F31 | RTV, PAA-8 (physical mixture) | 30: 70 |
| F32 | RTV, PAA-8 (physical mixture) | 50: 50 |

### 4. Evaluation of Products

The resulting ASDs and comparative examples are tested for stability at 40°C/75% RH and analyzed by: appearance, Differential Scanning Calorimetry (DSC), X-ray Powder Diffraction (XRPD) and drug dissolution. All drug-PAA ASDs prepared by spray drying show stability over time. Stabilization of high drug loading (80%, or higher) was achieved only for the linear PAA.

### A. Physical properties (Appearance, crystallinity, thermal behavior)

TABLE 8 shows physical properties of ITZ - PAA physical mixtures and spray dried amorphous solid dispersions. The PAA type shows the molecular weight designation and solvent (ethyl acetate: EA, ethyl acetate cyclohexane cosolvent: CO) used in formation of the PAA. The physical form of the product is identified visually (e.g., solid powder) and by XRPD and/or DSC (to assess amorphous or crystalline state). Tg values are estimates from DSC curves.

Results for the comparative polymers are shown in TABLE 9.

**TABLE 8: Physical properties of Formulations**

| Formu lation | Drug | PAA type | Drug: PAA (wt.ratio) | Process | Appearance | Form | Tg (°C) |
|---|---|---|---|---|---|---|---|
| F1 | ITZ | - | 100:0 | Spray dried | White powder | Amorphous* | 57 |
| F2 | ITZ | HMW-CO | 15:85 | Spray dried | White, cotton like | Amorphous | 125 |
| F3 | ITZ | HMW-CO | 30: 70 | Spray dried | White, cotton like | Amorphous | 130 |
| F4 | ITZ | HMW-CO | 40:60 | Spray dried | White, cotton like | Amorphous | 125 |
| F5 | ITZ | HMW-CO | 50:50 | Spray dried | White powder | Amorphous | 125 |
| F6 | ITZ, | LMW-CO | 40:60 | Spray dried | White, fibrous powder | Amorphous | 130 |
| F7 | ITZ | MMW-CO | 40:60 | Spray dried | White, fibrous fluffy powder | Amorphous | 125 |
| F8 | ITZ | MMW-EA | 40:60 | Spray dried | White fluffy powder | Amorphous | 130 |
| F9 | ITZ | MMW-EA | 70:30 | Spray dried | White powder | Amorphous | 100 |
| F10 | ITZ | MMW-EA | 80:20 | Spray dried | White powder | Amorphous | 95 |
| F11 | ITZ | HMW-EA | 80:20 | Spray dried | White powder | Amorphous | 90, 110 † |
| F12 | ITZ | MMW-EA | 90:10 | Spray dried | White static powder | Non-uniform amorphous system with an amorphous-amorphous phase separation †† | 75 |
| F13 | ITZ | LMW-EA | 80:20 | Spray dried | White powder | amorphous | 95 |
| F14 | ITZ | MMW-EA | 80:20 | Spray dried | White powder | amorphous | 95 |
| F15 | ITZ | MMW-EA | 80:20 | Spray dried | White powder | amorphous | 95 |
| F16 | RTV | -- | 100:0 | Spray dried | White powder | Amorphous* | 45 |
| F17 | RTV | HMW-CO | 15:85 | Spray dried | White, cotton like powder | Amorphous | ~ 115 |
| F18 | RTV | HMW-CO | 30:70 | Spray dried | White, cotton like powder | Amorphous | ~120 |
| F19 | RTV | HMW-CO | 50:50 | Spray dried | White, cotton like powder | Amorphous | ~115 |
| F24 | - | HMW-CO | 0:100 | Powder | White powder | Amorphous | 120 |
| F25 | ITZ | - | 100:0 | As purchased | White powder | Crystalline (no Tg) | N/A |
| F26 | ITZ | HMW-CO | 15:85 | Physical mixture | White powder | Crystalline + amorphous** | 120 |
| F27 | ITZ | HMW-CO | 30:70 | Physical mixture | White powder | Crystalline + amorphous** | 120 |
| F28 | ITZ | HMW-CO | 50:50 | Physical mixture | White powder | Crystalline + amorphous** | 120 |
| F29 | RTV | - | 100:0 | As purchased powder | White powder | Crystalline (Form II) No Tg | N/A |
| F30 | RTV | HMW-CO | 15:85 | Physical mixture | White powder | Crystalline + amorphous** | Overlap with API Tm |
| F31 | RTV | HMW-CO | 30:70 | Physical mixture | White powder | Crystalline + amorphous** | Overlap with API Tm |
| F32 | RTV | HMW-CO | 50:50 | Physical mixture | White powder | Crystalline + amorphous** | Overlap with API Tm |

| | | | | | | | |
|---|---|---|---|---|---|---|---|
| * Freshly spray dried ITZ and RTV are both amorphous. However, the product changes to a crystalline form very quickly upon storage. ** Crystalline pattern of neat drug + amorphous halo from PAA † ITZ melt observed †† DSC suggests phase separation. PXRD shows amorphous material. | | | | | | | |

The results suggest that API/linear PAA ASDs with drug loadings of up to 80 wt.% can be achieved by spray drying, without losing the amorphous character of the product. The API/linear PAA physical mixtures show crystallinity, attributable to the API.

Formulations F9 and F10 were placed in accelerated stability (40°C/75%RH) and analyzed at two weeks. This informal stability study showed no significant changes (still amorphous).

**TABLE 9: Physical properties of ITZ - Polymer (Soluplus^{®}; Affinisol^{®}) spray dried solid dispersions**

| Formulation | Polymer | ITZ: Polymer weight ratio | Appearance | Form | Tg (°C) |
|---|---|---|---|---|---|
| F20 | Soluplus^{®} | 40:60 | White, free flowing powder | amorphous | ND |
| F21 | Soluplus^{®} | 80:20 | White static powder | Non-uniform amorphous system with an amorphous - amorphous phase separation * | 50 |
| F22 | Affinisol^{®} | 40:60 | White, free flowing powder | amorphous | ND |
| F23 | Affinisol^{®} | 80:20 | White static powder | Non-uniform amorphous system with an amorphous - amorphous phase separation * | ND |

| | | | | | |
|---|---|---|---|---|---|
| *ITZ melt observed; Recrystallization and melting endotherm. DSC suggests phase separation. PXRD shows amorphous material. ND: Could not reliably be determined from existing data | | | | | |

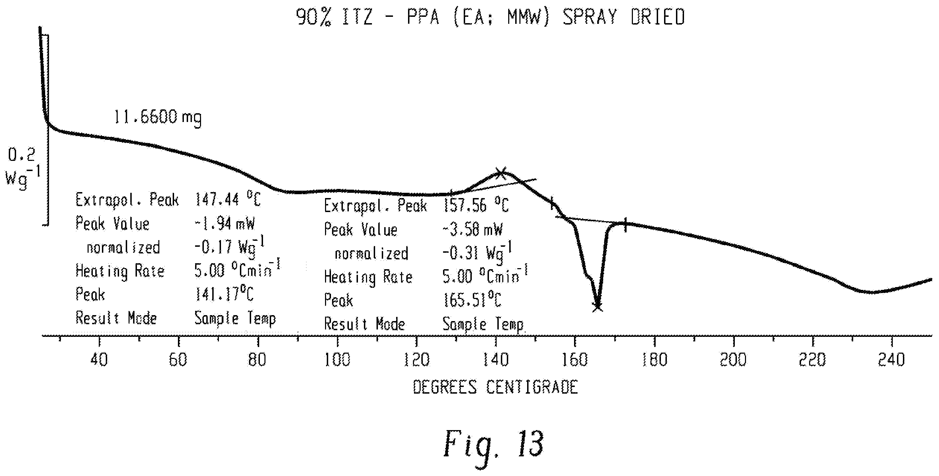

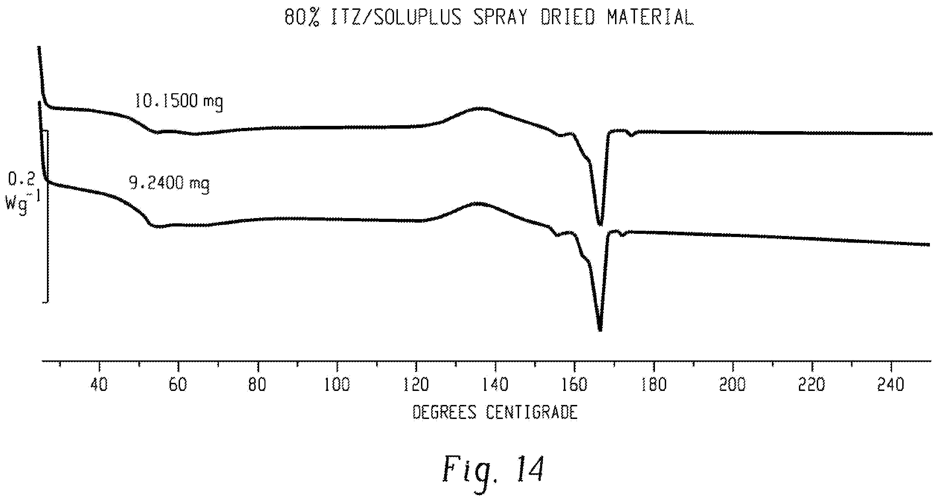

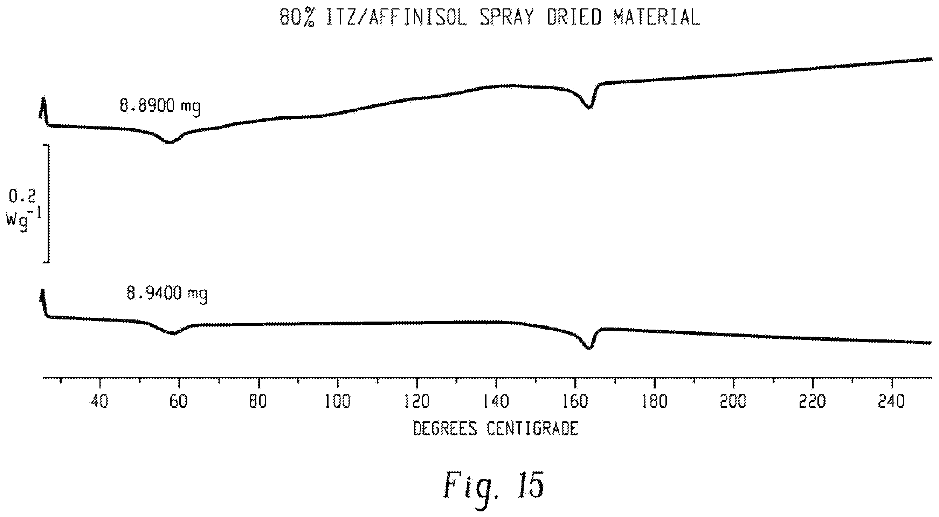

In the case of 80% ITZ Soluplus^{®} or Affinisol^{®} spray dried materials (F21 and F23, Table 9), XRPD shows an amorphous system, while the DSC shows broader features with a melting peak characteristic for crystalline ITZ (Figure 14 and 15). These two results combined support the conclusion that a non-uniform amorphous system with an amorphous - amorphous phase separation comprising ITZ amorphous drug occurs in the 80% ITZ Soluplus^{®} or Affinisol^{®} spray dried materials. The same features are present in XRPD and DSC of the spray dried material containing 90%ITZ and PAA (F12; Table 8; Figure 13).

These results suggest that at high drug loading, spray dried ASD cannot be achieved with Soluplus^{®} and Affinisol^{®} polymers without phase separation of the drug. From a physical stability standpoint, phase separation is undesirable as it impacts drug dissolution and long-term stability, with increase potential for drug crystallization in time.

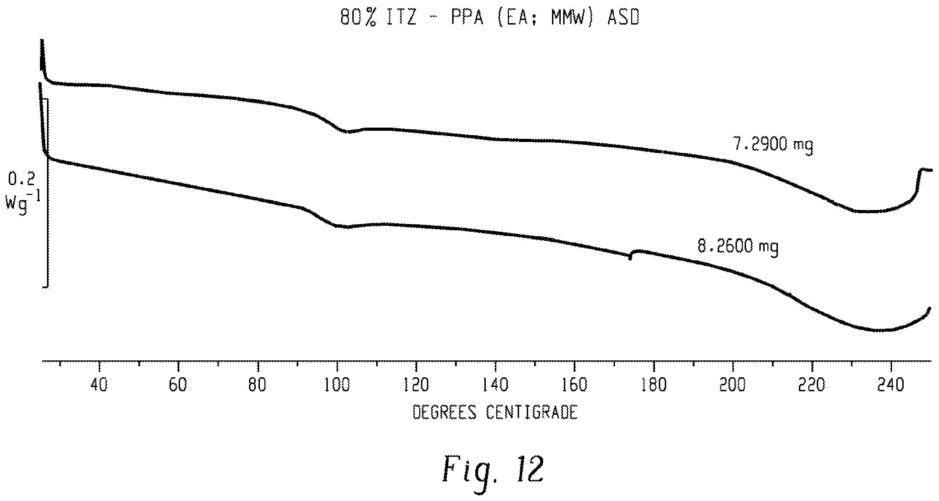

For 80% ITZ-PAA amorphous solid dispersion, DSC shows no melting peak characteristic of crystalline ITZ (FIG. 12) and the XRPD shows an amorphous material. These results indicate that 80%ITZ-PAA is a uniform, single phase amorphous solid dispersion with no drug phase separation. Thus, PAA is a more effective polymer in maintaining physical stability of drug-polymer ASDs at high drug loading levels than the benchmarks Soluplus^{®} and Affinisol^{®}.

FIGURES 2-7 are photographs of spray-dried products at the same scale. FIGURE 2 shows product F20 (40% ITZ - 60% Soluplus^{®}); FIGURE 3 shows product F22 (40% ITZ - 60% Affinisol^{®}); FIGURE 4 shows product F4 (40% ITZ - 60% PAA (HMW-CO)); FIGURE 5 shows product F7 (40% ITZ - 60% PAA (MMW-CO)); FIGURE 6 shows product F6 (40% ITZ - 60% PAA (LMW-CO)); and FIGURE 7 shows product F8 (40% ITZ - 60% PAA (MMW-EA)).

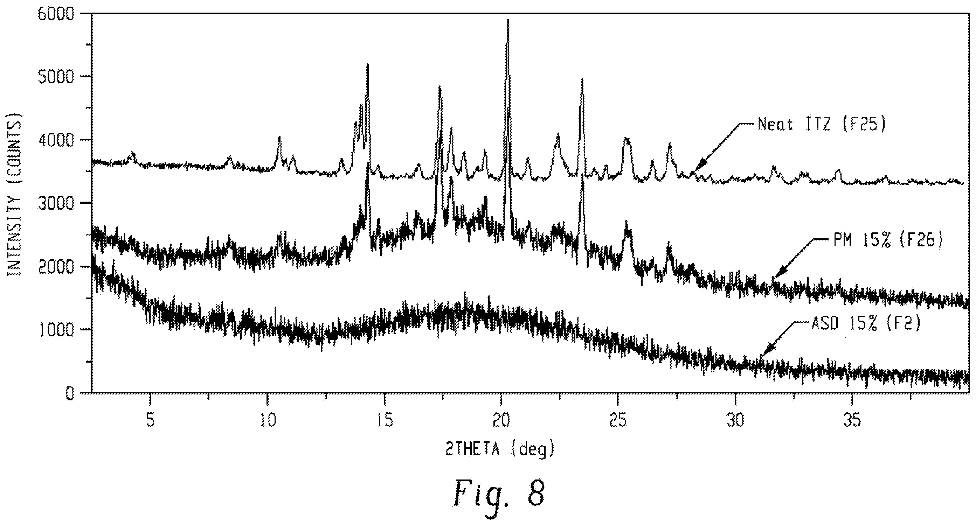

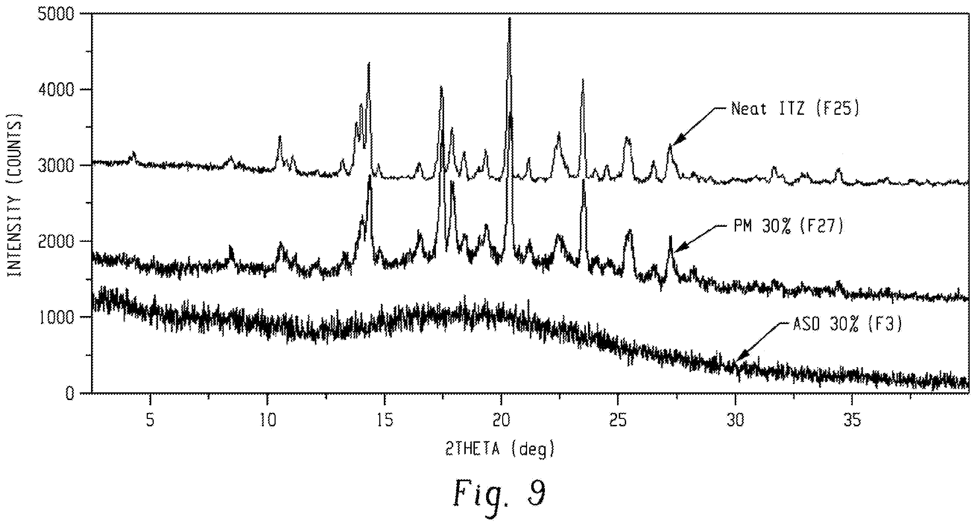

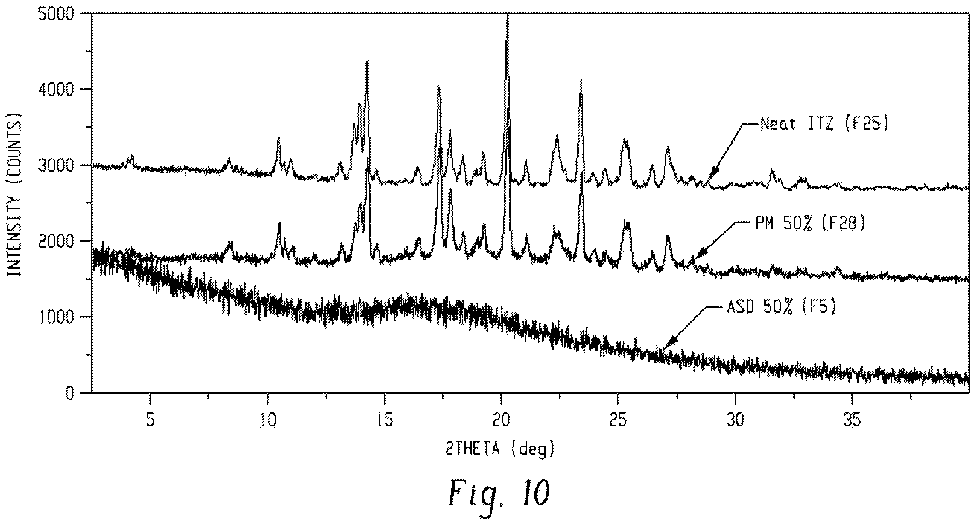

X-ray Powder Diffraction (XRPD) is performed with a Panalytical X'Pert³ Powder XRPD. FIGURES 8-10 show XRPD plots for various formulations made using ITZ and PAA-8. In FIG. 8, plots for ITZ alone (Neat IZT, Formulation F25), a physical mixture of 15% ITZ and 85% PAA (PM 15%, Formulation F26), and a spray-dried solid dispersion of 15% ITZ and 85% PAA (ASD 15%, Formulation F2), are shown. In FIG. 9, plots for ITZ alone (Neat IZT, Formulation F25), a physical mixture of 30% ITZ and 70% PAA (PM 30%, Formulation F27), and a spray-dried solid dispersion of 30% ITZ and 70% PAA (ASD 30%, Formulation F3), are shown. In FIG. 10, plots for ITZ alone (Neat IZT, Formulation F25), a physical mixture of 50% ITZ and 50% PAA (PM 50%, Formulation F28), and a spray-dried solid dispersion of 50% ITZ and 50% PAA (ASD 50%, Formulation F5), are shown.

The plots indicate that the exemplary ASDs are substantially amorphous (no large peaks in the spectrum), even at high drug loading (50% ITZ). In contrast, both the neat ITZ and physical mixtures show significant crystalline character, as evidenced by the large peaks.

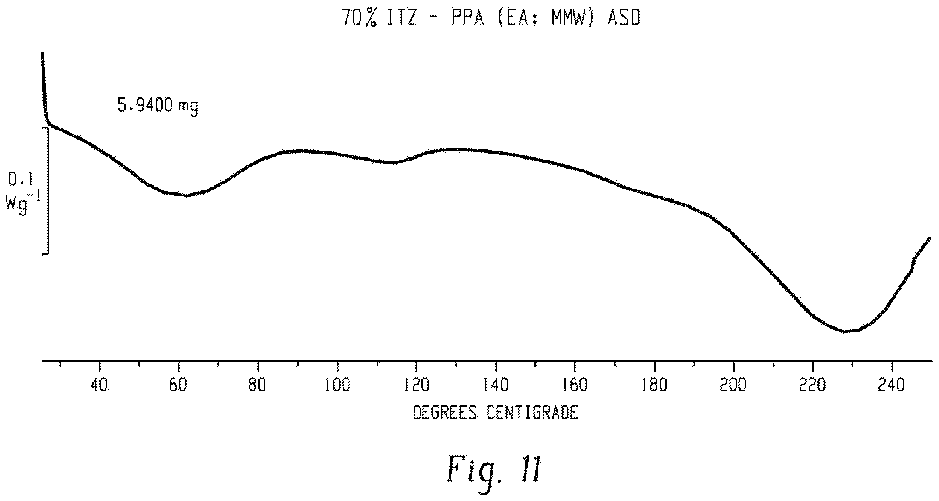

FIGURES 11-13 show DSC plots for Formulations F9 (spray dried ASD, 70% ITZ-30% PAA (MMW-EA)), F10 (spray dried ASD, 80% ITZ-20% PAA (MMW-EA)) and F12 (spray dried 90% ITZ-10% PAA (MMW-EA)), respectively. FIGURES 14 and 15 show DSC plots for Formulation F21 (spray dried 80% ITZ 20% Soluplus^{®}) and Formulation F23 (spray dried 80% ITZ-20% Affinisol^{®}) materials, respectively. The DSC plots show that when Affinisol^{®} or Soluplus^{®} polymers are spray dried with 80% drug, ITZ recrystallization and melting point peaks are observed. These peaks are absent when linear PAA is spray dried with 80% API.

### B. Assay and drug recovery from spray dried ASDs

To evaluate the drug content of the formulations, 25 mg of a formulated sample (equivalent to 10 mg API) is added to a 50 mL volumetric flask. 5 mL of solvent (1:2 DCM:ethanol, by volume) is added to dissolve the sample and the mixture is briefly sonicated. Each sample is brought to the volume of the flask with diluent (70:30 Methanol:0.1N HCl, by volume), and mixed well. The mixture is analyzed by HPLC using the Assay/Related Substance method A Waters Alliance HPLC is used. TABLE 10 shows the results of the ITZ assay.

**TABLE 10: ITZ assay**

| | ITZ Assay % Recovery (n = 3) | | |
|---|---|---|---|
| Formulation | F4 40% ITZ - 60% PAA-8 (CO-HMW) | F7 40% ITZ- 60% PAA-7 (CO-MMW) | F6 60% ITZ - 40% PAA-6 (CO-LMW) |
| Recovery (average over 3 samples) | 94 | 92 | 92 |

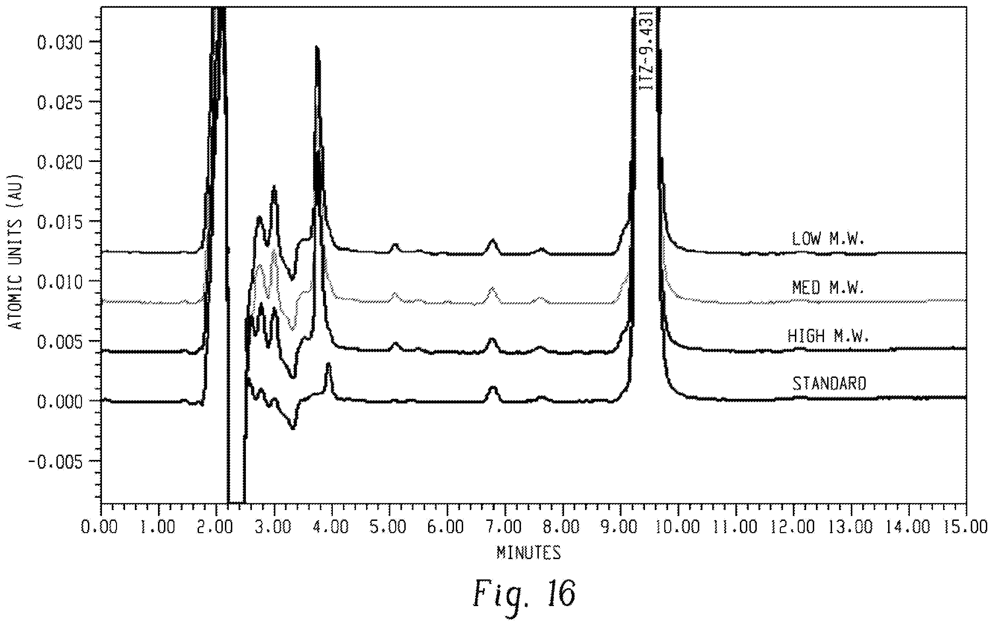

FIGURE 16 shows chromatograms of the ITZ assay of Formulations F4, F7, and F6.

### C. Drug dissolution testing

The results of drug dissolution testing indicate that the model drugs are more effectively released from the spray dried linear PAA ASDs than from dispersions made with conventional polymers and from physical mixtures. For these tests a dissolution bath is used (Distek Model 6100 or 7100).

### i) Itraconazole: 15%, 30%, 50%, by weight, ITZ-Linear PAA ASDs

The method is performed under non-sink conditions. The solubility limit of ITZ in an equilibrated dissolution media (750 mL 0.1N HCl, 37°C) is about 4-6 µg/mL and the in-vessel ITZ concentration is about 50 µg/mL.

Product equivalent to about 37.5 mg of itraconazole is weighed into a 50mL plastic centrifuge tube (i.e., for 15% loading = 250 mg, for 30% loading = 125 mg, and for 50% loading = 75 mg). 40 mL of equilibrated media is removed from a vessel containing 750 mL. About 10 mL of the removed equilibrated media is added to the tube vial to pre-wet the drug. The tube is shaken by hand, and the contents transferred to the vessel. The tube is rinsed with the remaining portion of the 40 mL media and the contents returned to the vessel. Apparatus II (paddles) @ 75 rpm, is used to mix the sample.

Samples (3 each) are removed from the vessel at 5, 10, 15, 30, 45, 60, and 120 minutes. The sampling is performed using 10 µm cannula tip filters and 0.2 µm regenerated cellulose (Thermo F2513-8) filters for post sample collection. To take a sample, the cannula is purged 1-2 times before collecting 5mL into a disposable syringe. The disposable syringe is removed from the cannula and the cellulose filter fitted to the syringe. The filter is flushed with ~4mL of collected sample, back into vessel. The remaining 1mL of the sample is collected in a glass vial. The collected sample is diluted 1:1 with ACN by transferring 750 µL of the sample to an HPLC vial and adding 750 µL of ACN. A vortex is used to mix the diluted sample and HPLC analysis is performed.

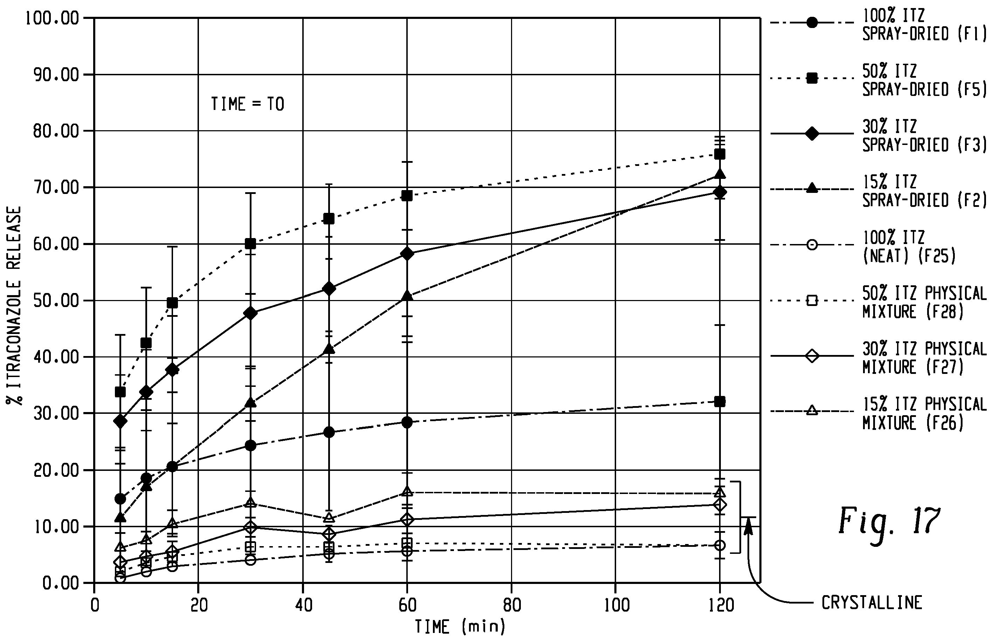

FIGURE 17 shows release of ITZ (mean of 3 samples) in 0.1N HCl under non-sink conditions from ITZ - PAA (co-solvent) physical mixtures (PM) (Formulations F26, F27, and F28), spray dried (SD) ASDs (Formulations F2, F3, and F5), ITZ neat (Formulation F25), and ITZ SD (Formulation F1).

### ii) Itraconazole - 40% ITZ-60% Linear PAA Polymer ASD, Itraconazole - 40% ITZ-60% polymer (Soluplus^{®} or Affinisol^{®}) dispersions, and Itraconazole - 80% - 20% Linear PAA Polymer ASD

The method is performed under non-sink conditions. Product equivalent to 100 mg ITZ (40% loading = 250 mg; 80% loading = 125 mg) is added to a centrifuge. Immediately prior to dissolution, about 40mL of equilibrated media is removed from the respective vessel and a small amount (~10mL) is added to the tube vial to pre-wet the product, which is shaken by hand, and transferred to the vessel. This is repeated with remaining media so that all media originally removed from the vessel is returned to the vessel. Two equilibrated dissolution media are used: 900 mL of pH 6.8 of phosphate buffer at 37°C and 900 mL of 0.1N HCl 37°C. Apparatus II (paddles) @ 75 rpm, is used to mix the sample.

Samples (3 each) are removed from the vessel at 5, 10, 15, 30, 45, 60, and 120 minutes, as described for i) above.

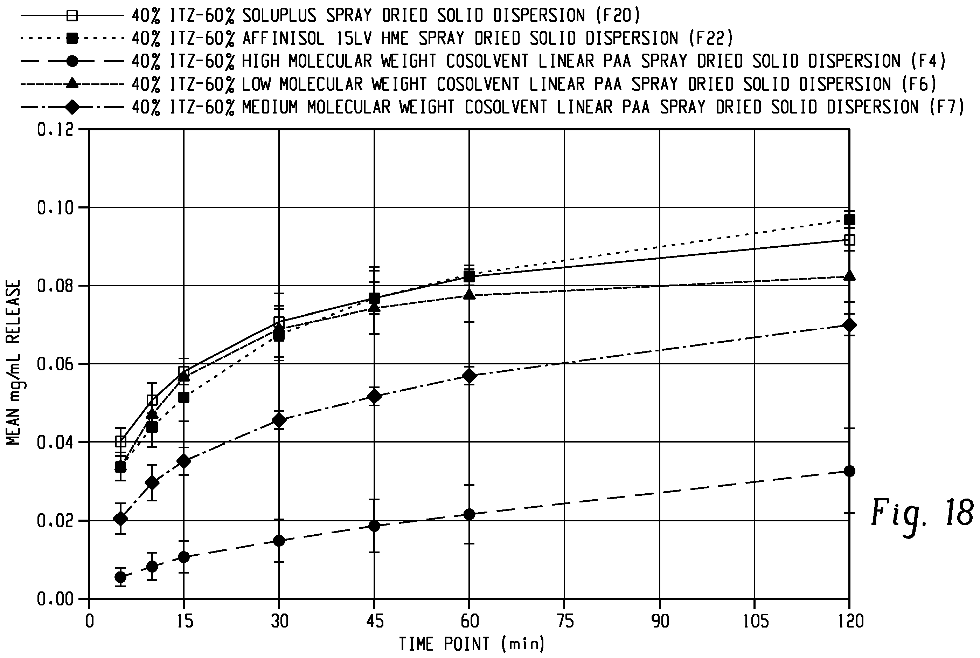

FIGURE 18 shows ITZ release (mean of 3 samples) under non-sink conditions in 0.1N HCl from 40% ITZ - 60% PAA (Formulations F4: co-solvent HMW; F6: co-solvent, LMW; and F7: cosolvent, MMW) spray dried ASDs, as well as 40% ITZ - 60% Soluplus^{®} ASD (Formulation F20) and 40% ITZ - 60% Affinisol^{®} ASD (Formulation F22).

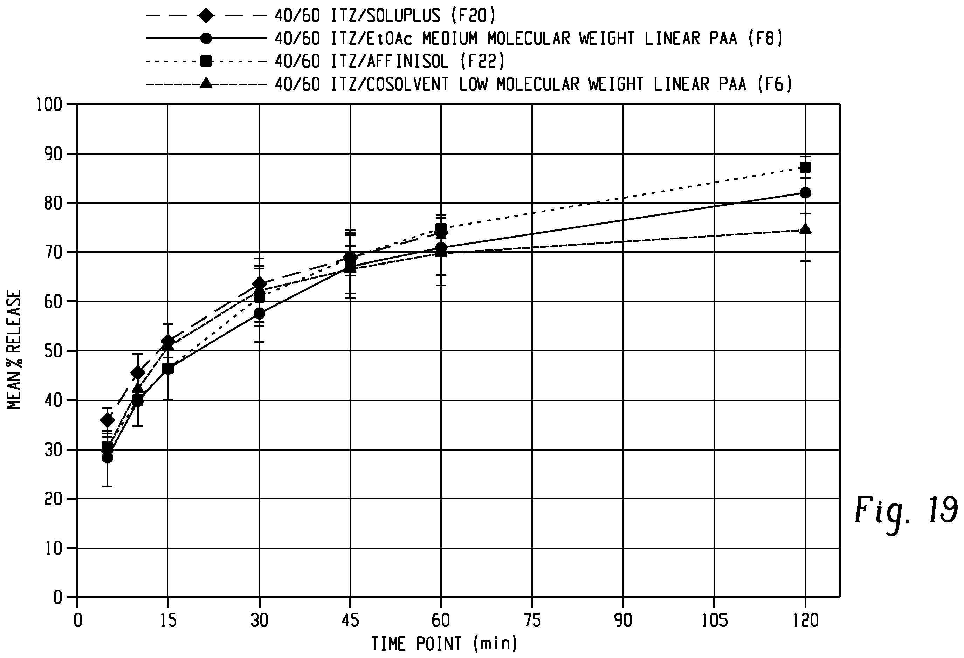

FIGURE 19 shows mean ITZ release (of 3 samples) under non-sink conditions in 0.1N HCl from 40% ITZ - 60% PAA (Formulation F8: ethyl acetate; MMW) spray dried ASD, 40% ITZ - 60% Soluplus^{®} ASD (Formulation F20), 40% ITZ - 60% Affinisol^{®} ASD (Formulation F22), and 40% ITZ - 60% PAA ASD (Formulation F6).

### iii) Ritonavir - 15%, 30% and 50% RTV-Linear PAA ASD

The method is performed under non-sink conditions. The solubility limit of RTZ in an equilibrated dissolution media (of pH 6.8 phosphate buffer 37°C) is about 1 µg/mL and the in-vessel ITZ concentration is about 13 µg/mL.

Product equivalent to about 10 mg of ritonavir is weighed into a 50mL plastic centrifuge tube (i.e., for 15% loading = 66.66 mg, for 30% loading = 33.33 mg, and for 50% loading = 20 mg). Immediately prior to dissolution, 40 mL of equilibrated media is removed from a vessel containing 750 mL. About 10 mL of the removed equilibrated media is added to the tube vial to pre-wet the drug. The tube is shaken by hand, and the contents transferred to the vessel. The tube is rinsed with the remaining portion of the 40 mL media and the contents returned to the vessel. Apparatus II (paddles) @ 75 rpm, is used to mix the sample.

Samples (3 each) are removed from the vessel at 5, 10, 15, 30, 45, 60, and 120 minutes. The sampling is performed using 10 µm cannula tip filters and 0.45 µm PVDF w/GMF (Whatman Cat# 6872-2504) filters for post sample collection, as described for i) above.

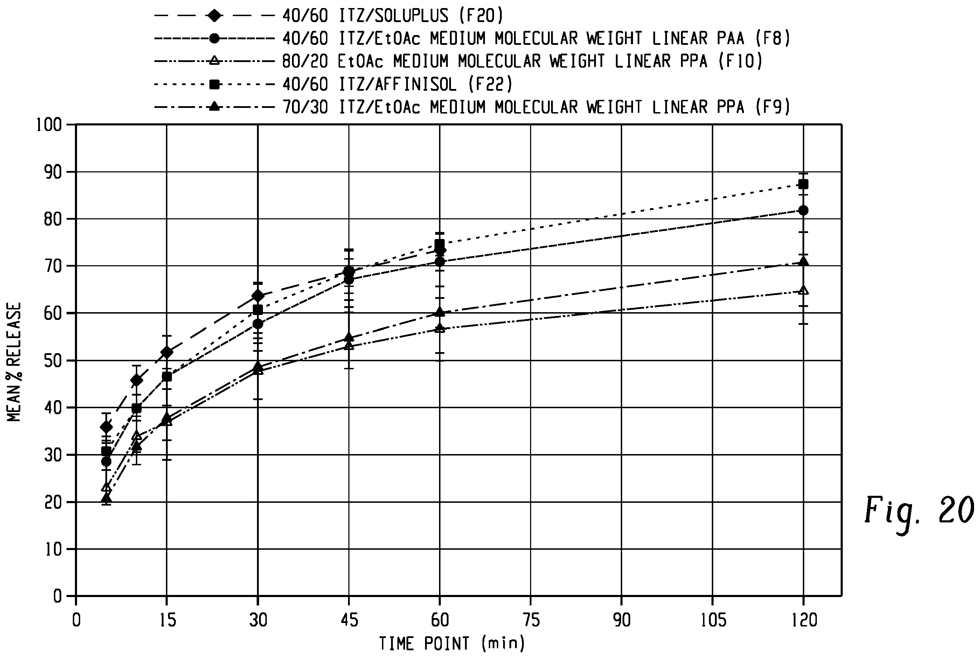

FIGURE 20 shows ITZ release (mean of 3 samples) under non-sink conditions in 0.1N HCl from 80% ITZ - 20% PAA spray dried ASD (Formulation F10: ethyl acetate MMW PAA); 40% ITZ - 60% PAA spray dried ASD (Formulation F8: ethyl acetate MMW PAA); 70% ITZ -30% PAA spray dried ASD (Formulation F9: ethyl acetate MMW PAA); and 40% ITZ - 60% Soluplus^{®} and 40% ITZ - 60% Affinisol^{®} spray dried ASDs (Formulations F20 and F22). Soluplus^{®} and Affinisol^{®} were not suitable to prepare ASDs at 80% drug loading.

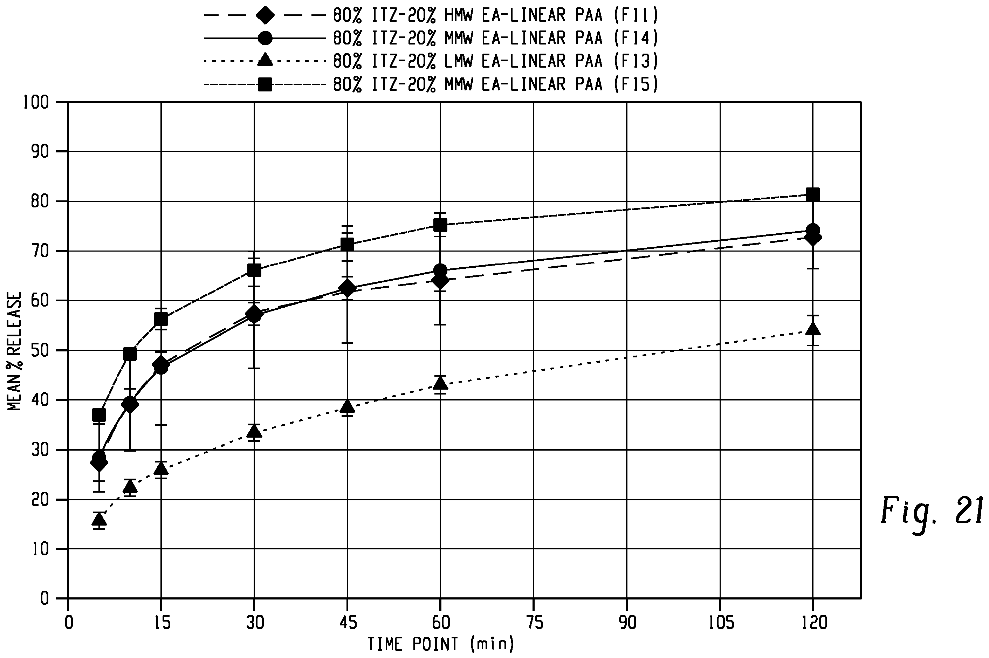

FIGURE 21 shows ITZ release (mean of 3 samples) under non-sink conditions in 0.1N HCl from 80% ITZ - 20% PAA spray dried ASDs (Formulation F13: ethyl acetate LMW PAA, Formulation F14: ethyl acetate MMW PAA, Formulation F15: ethyl acetate MMW PAA, and Formulation F11: ethyl acetate HMW PAA).

### D. Stability study

Product samples are stored in 1 oz. (~28 gm) glass jars with screw tops for the duration of the study. For itraconazole, approximately 0.4-1.1 g of sample per jar is used. For ritonavir, approximately 0.3 - 0.5 g is used. The containers are stored at 40-45°C/75% RH in a stability chamber (Caron 7000-50-1, Darwin Chambers ICH-G2HD-11X11) and tested at T0, 1 month, 2 months, 3 months, and in some cases up to 6 months. The tests performed include appearance, dissolution, DSC, and XRPD.

### XRPD

XRPD is performed with Si zero background holders. The 2-theta position is performed with a Panalytical Si reference standard disc. The XRPD instrument configuration is Bragg-Brentano geometry. TABLE 11 shows the parameters used.

**TABLE 11: XRPD Parameters**

| **Parameters** | **Reflection Mode** |
|---|---|
| X-Ray wavelength | Cu, kαKα1 (Å): 1.540598, Kα2 (Å): 1.544426, Kα2/Kα1 intensity ratio: 0.50 |
| X-Ray tube setting | 45 kV, 40 mA |
| Divergence slit | Fixed 1/8° |
| Scan mode | Continuous |
| Scan range (° 2TH) | 3-40 |
| Scan step time (s) | 18.87 |
| Step size (° 2TH) | 0.0131 |
| Test Time | 4 min 15 s |

### DSC

Differential Scanning calorimetry (DSC) is performed with a Mettler-Toldeo DSC-1 instrument (no modulated DSC software) using a sample amount of 5-10mg. The pan type is aluminum, 40µL; contents crimped, lid pierced. The sample is heated in the pan from 25-250 °C, increasing at 5 deg/min under a nitrogen purge. Temperature and heat of fusion are calibrated with an appropriate reference material (indium).

ITZ - PAA (co-solvent) samples at 15%, 30%, 50% and 100% ITZ are tested at 40°C/75% RH. Both XRPD and DSC show that the spray dried formulations of 100% ITZ converted from amorphous to crystalline form at one month and no further form change was observed at two months, and three months. All spray dried formulations containing ITZ and PAA remained amorphous for the duration of the study. Dissolution data support the observations made by XRPD and DSC. 30% and 50% ITZ-PAA ASDs showed no meaningful change of dissolution patterns. The 15% ITZ - PAA ASD showed a decrease in drug release after three months, however the XRPD and DSC showed amorphous state was maintained.

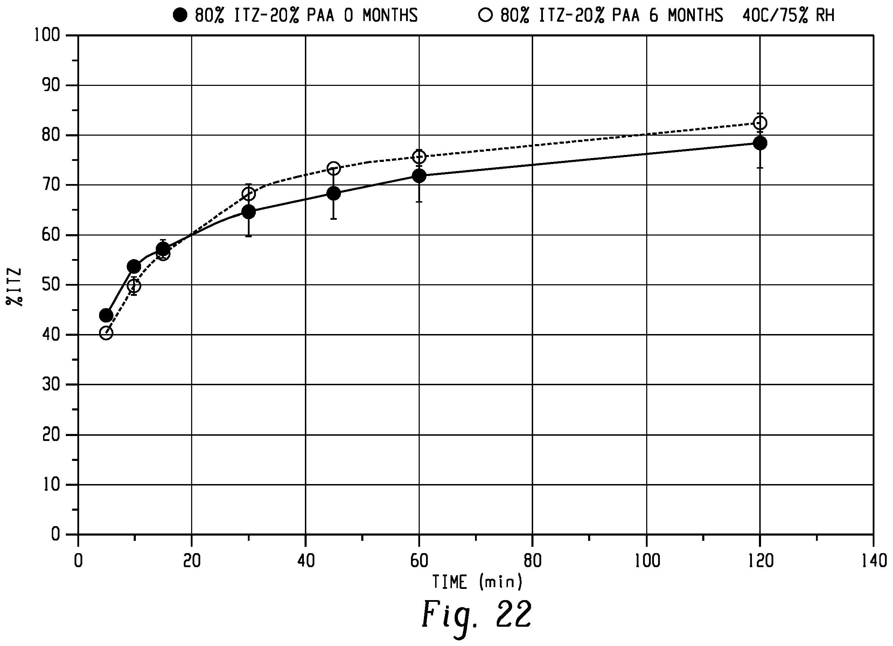

Samples of 40%, 60% and 80% ITZ - PAA (MMW-EA) spray dried ASDs are tested in accelerated stability conditions (40 °C/75%RH) for 6 months. Both XRPD and DSC show that all spray dried formulations containing ITZ and PAA remained amorphous for the duration of the study. Dissolution data support the observations made by XRPD and DSC showing no meaningful reduction in dissolution rate (see for example 80% ITZ-PAA ASDs, FIGURE 22).

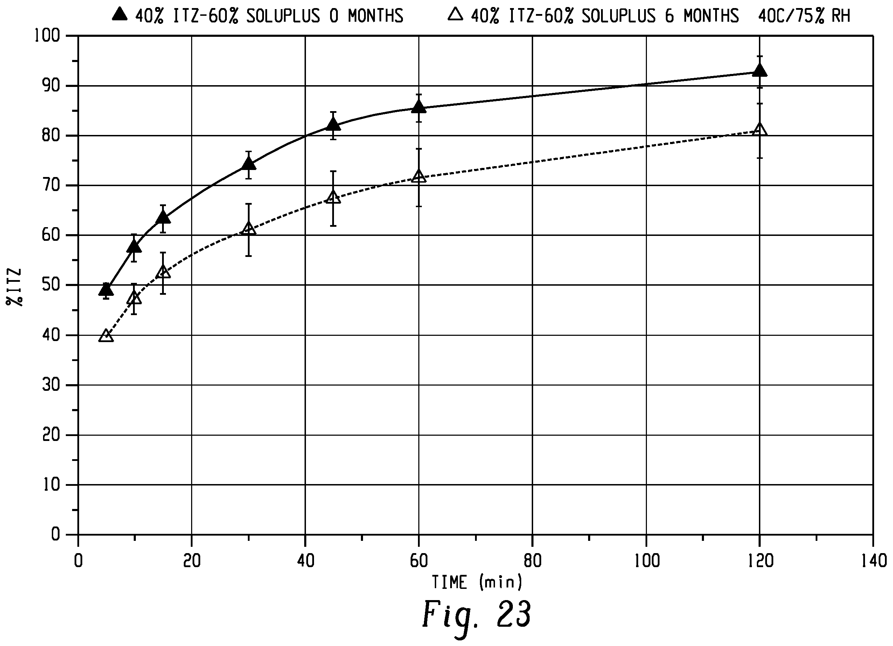

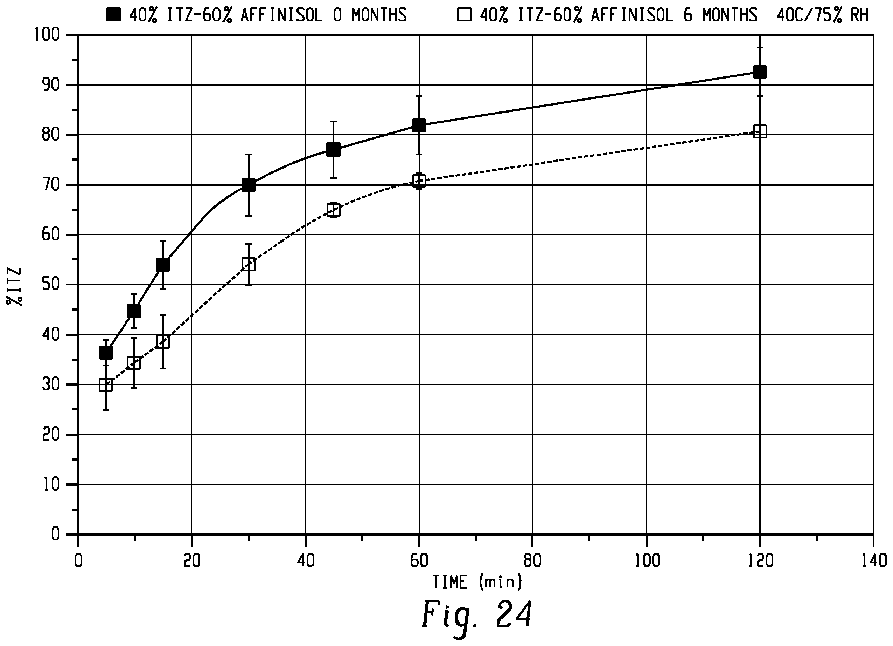

Samples of 40%ITZ -Soluplus and 40% ITZ-Affinisol^{®} spray dried ASDs tested in accelerated stability conditions (40 °C/75%RH) for 6 months show they remain amorphous for the duration of study; however, a decrease of drug release was observed for the 6 months' time point (FIGURE 23; FIGURE 24).

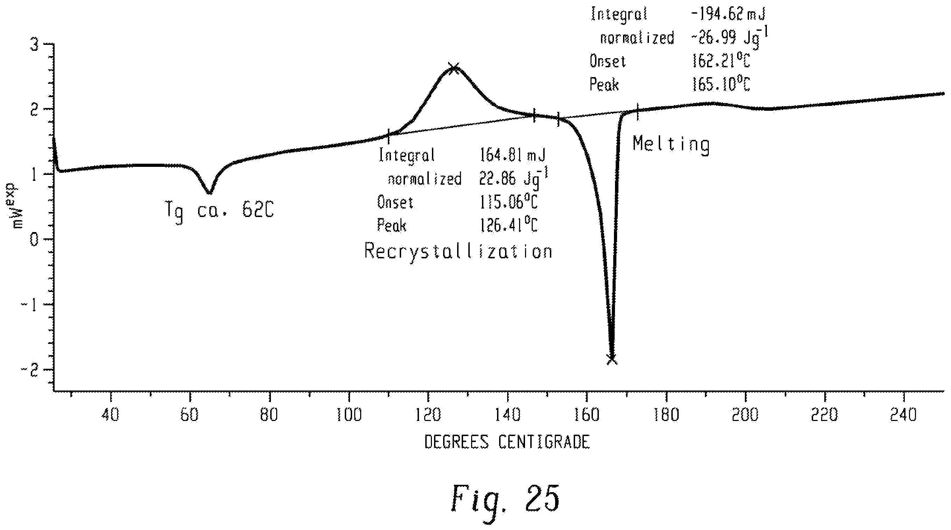

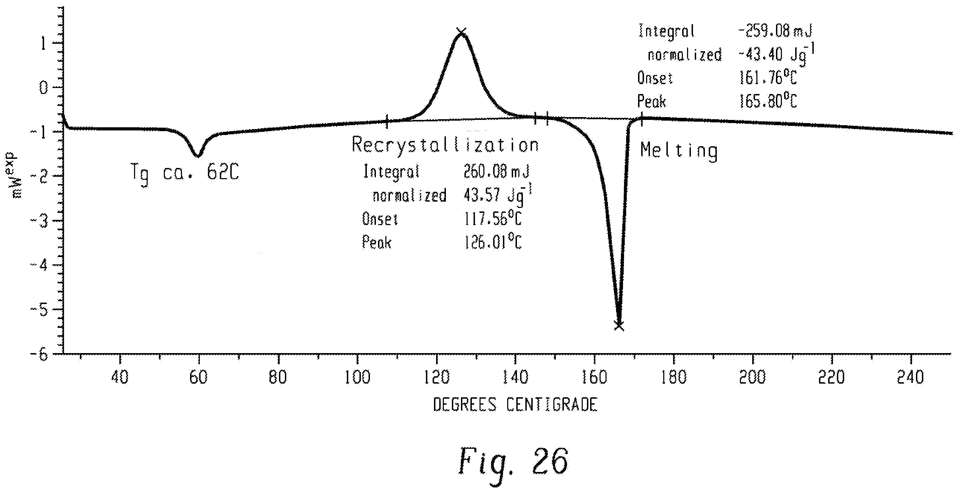

As prepared spray dried 80% ITZ - Soluplus^{®} and 80% ITZ- Affinisol^{®} materials are non-uniform amorphous systems with an amorphous - amorphous phase separation comprising ITZ amorphous drug (FIGURES 14 and 15; Table 9). This may be undesirable for some applications as it impacts long-term stability, with increase potential for drug crystallization in time. Storage of spray dried 80% ITZ-Soluplus^{®} and 80% ITZ - Affinisol^{®} materials at 40°C/75%RH for 3 months affected their thermal behavior with increased recrystallization on heating (FIGURE 25; FIGURE 26).

RTV - PAA (co-solvent) samples at 15%, 30%, 50% and 100% are tested at 45°C/75% RH. Both XRPD and DSC show that spray dried formulations of 100% RTV converted from amorphous to crystalline form I at two months and that no form change is observed at three months. All spray dried formulations containing RTV and PAA remain amorphous for the duration of the study. Dissolution data support the observations made by XRPD and DSC. 15% and 30% RTV-PAA ASDs show no meaningful change of dissolution patterns. The 50% RTV - PAA ASD show a decrease in drug release over two months, but no change between two and three months. However, XRPD and DSC show that the amorphous state is maintained.

The reduced dissolution observed for 15% ITZ-PAA ASD and 50% RTV-PAA may be associated with a "clumping" of the material upon storage. Change in surface area may have an impact in initial wettability of the powder, which can lead to the changes in dissolution profile.

Except in the Examples, or where otherwise explicitly indicated, all numerical quantities in this description specifying amounts of materials, reaction conditions, molecular weights, number of carbon atoms, and the like, are to be understood as modified by the word "about." Unless otherwise indicated, each chemical or composition referred to herein should be interpreted as being a commercial grade material which may contain the isomers, by-products, derivatives, and other such materials which are normally understood to be present in the commercial grade. However, the amount of each chemical component is presented exclusive of any solvent or diluent oil, which may be customarily present In the commercial material, unless otherwise indicated. It is to be understood that the upper and lower amount, range, and ratio limits set forth herein may be independently combined. Similarly, the ranges and amounts for each element of the invention may be used together with ranges or amounts for any of the other elements.

## Claims

1. An amorphous solid dispersion comprising:
at least 10 wt.% of a linear poly(acrylic acid); and
an active pharmaceutical ingredient,
wherein:
the linear poly(acrylic acid) has a Brookfield viscosity at 25°C of at least 100 mPa·s (at least 100 cP),
the linear poly(acrylic acid) and the active pharmaceutical ingredient together comprise at least 80 wt.% of the amorphous solid dispersion, and
the linear poly(acrylic acid) has a weight average molecular weight, determined as described in the description, by size exclusion chromatography on a liquid sample of 1.5 g/L polymer in 0.1M NaNO₃ at pH 10, of at least 200,000 g/mol (at least 200,000 Da), or at least 250,000 g/mol (at least 250,000 Da), or at least 300,000 g/mol (at least 300,000 Da), or at least 400,000 g/mol (at least 400,000 Da), or at least 500,000 g/mol (at least 500,000 Da), or at least 600,000 g/mol (at least 600,000 Da), or at least 800,000 g/mol (at least 800,000 Da), or at least 1,000,000 g/mol (at least 1,000,000 Da).

2. The amorphous solid dispersion of claim 1, wherein a ratio by weight of active pharmaceutical ingredient: poly(acrylic acid) in the amorphous solid dispersion is at least 1:10, or at least 1:6, or at least 1:3, or at least 1:1.5, or at least 1:1, or at least 2:1, or at least 3:1, or at least 4:1, or up to 6:1, or up to 5:1, or up to 4.5:1.

3. The amorphous solid dispersion of claim 1 or 2, wherein the Brookfield viscosity at 25°C of the linear poly(acrylic acid) is at least 200 mPa·s (at least 200 cP), or at least 250 mPa·s (at least 250 cP), or at least 300 mPa·s (at least 300 cP), or at least 400 mPa·s (at least 400 cP), and/or
no more than 3000 mPa·s (no more than 3000 cP), or no more than 2500 mPa·s (no more than 2,500 cP), or no more than 2200 mPa·s (no more than 2200 cP), or no more than 2100 mPa·s (no more than 2100 cP).

4. The amorphous solid dispersion of any one of claims 1-3, wherein the amorphous solid dispersion comprises at least 15 wt.% linear poly(acrylic acid), or at least 20 wt.% linear poly(acrylic acid), or at least 25 wt.% linear poly(acrylic acid), and/or
no more than 95 wt.% linear poly(acrylic acid), or no more than 80 wt.% linear poly(acrylic acid), or no more than 60 wt.% linear poly(acrylic acid), or no more than 50 wt.% linear poly(acrylic acid), or no more than 40 wt.% linear poly(acrylic acid), or no more than 30 wt.% linear poly(acrylic acid).

5. The amorphous solid dispersion of any one of claims 1-4, wherein the linear poly(acrylic acid) and the active pharmaceutical ingredient together comprise at least 90 wt.%, or at least 95 wt.% of the amorphous solid dispersion.

6. The amorphous solid dispersion of any one of claims 1-5, wherein the amorphous solid dispersion comprises no more than 10 wt. % water, or no more than 5 wt. % water, or no more than 1 wt. % water, or no water.

7. The amorphous solid dispersion of any one of claims 1-6, wherein the active pharmaceutical ingredient is in BCS class II or BCS class IV.

8. A product comprising the amorphous solid dispersion of any one of claims 1-7 and, optionally
at least one excipient or adjuvant.

9. The product of claim 8, wherein the product is in a form selected from the group consisting of granules, capsules, pellets, tablets, films, and implants.

10. A method of forming an amorphous solid dispersion of an active pharmaceutical ingredient, the method comprising:
forming a liquid dispersion comprising a linear poly(acrylic acid), an active pharmaceutical ingredient, and a solvent system, the linear poly(acrylic acid) having a Brookfield viscosity at 25°C of at least 100 mPa·s (at least 100 cP) and a weight average molecular weight, determined as described in the description, by size exclusion chromatography on a liquid sample of 1.5 g/L polymer in 0.1M NaNO₃ at pH 10, of at least 200,000 g/mol (at least 200,000 Da), or at least 250,000 g/mol (at least 250,000 Da), or at least 300,000 g/mol (at least 300,000 Da), or at least 400,000 g/mol (at least 400,000 Da), or at least 500,000 g/mol (at least 500,000 Da), or at least 600,000 g/mol (at least 600,000 Da), or at least 800,000 g/mol (at least 800,000 Da), or at least 1,000,000 g/mol (at least 1,000,000 Da); and
evaporating the solvent system from the liquid dispersion to form an amorphous solid dispersion,
wherein the amorphous solid dispersion comprises at least 10 wt.% linear poly(acrylic acid) and wherein the linear poly(acrylic acid) and the active pharmaceutical ingredient together comprise at least 80 wt.% of the amorphous solid dispersion.

11. The method of claim 10, wherein a weight ratio of active pharmaceutical ingredient: linear poly(acrylic acid) in the liquid dispersion is at least 15:85, or at least 30:70, or at least 40:60, or at least 50:50, or at least 70:30 and/or
wherein a weight ratio of active pharmaceutical ingredient: linear poly(acrylic acid) in the liquid dispersion is no more than 90:10, or no more than 85:15.

12. The method of any one of claims 10-11, wherein the linear poly(acrylic acid) is one which has been formed in a solvent system which is substantially free of water.

13. The method of any one of claims 10-12, wherein the linear poly(acrylic acid) is one which has been formed in a solvent system selected from a) ethyl acetate and b) a mixture of ethyl acetate and cyclohexane.

14. The method of any one of claims 10-13, wherein the forming of the dispersion of the linear poly(acrylic acid) and the active pharmaceutical ingredient comprises dissolving the linear poly(acrylic acid), in powder form in the solvent system or in at least one of a plurality of solvents used in the solvent system, and/or
wherein the solvent system comprises at least one of an organic polar protic solvent and a polar aprotic solvent, in particular wherein the solvent system comprises at least one organic polar protic solvent selected from C₁-C₆ alcohols, and mixtures thereof, and/or wherein the solvent system comprises at least one polar aprotic solvent selected from dichloromethane, C₃-C₈ ketones, C₃-C₈ ethers, and mixtures thereof.

15. Use of the amorphous solid dispersion of any one of claims 1-7 or the product of any one of claims 8-9, or the amorphous solid dispersion formed by the method of any one of claims 10-14, or a product comprising the amorphous solid dispersion formed by the method of any one of claims 10-14 for administering an active pharmaceutical ingredient.

## Patentansprüche

1. Amorphe, feste Dispersion, umfassend:
zu mindestens 10 Gew.-% eine lineare Poly(acrylsäure); und
einen pharmazeutischen Wirkstoff,
wobei:
die lineare Poly(acrylsäure) eine Brookfield-Viskosität bei 25 °C von mindestens 100 mPa·s (mindestens 100 cP) aufweist,
die lineare Poly(acrylsäure) und der pharmazeutische Wirkstoff zusammen mindestens 80 Gew.-% der amorphen, festen Dispersion umfassen, und
die lineare Poly(acrylsäure) ein gewichtsmittleres Molekulargewicht, bestimmt wie in der Beschreibung beschrieben, durch Größenausschlusschromatographie an einer flüssigen Probe von 1,5 g/l Polymer in 0,1 M NaNO₃ bei pH 10, von mindestens 200.000 g/mol (mindestens 200.000 Da) oder mindestens 250.000 g/mol (mindestens 250.000 Da) oder mindestens 300.000 g/mol (mindestens 300.000 Da) oder mindestens 400.000 g/mol (mindestens 400.000 Da) oder mindestens 500.000 g/mol (mindestens 500.000 Da) oder mindestens 600.000 g/mol (mindestens 600.000 Da) oder mindestens 800.000 g/mol (mindestens 800.000 Da) oder mindestens 1.000.000 g/mol (mindestens 1.000.000 Da) aufweist.

2. Amorphe, feste Dispersion nach Anspruch 1, wobei ein Gewichtsverhältnis von pharmazeutischem Wirkstoff:Poly(acrylsäure) in der amorphen, festen Dispersion mindestens 1:10 oder mindestens 1:6 oder mindestens 1:3 oder mindestens 1:1,5 oder mindestens 1:1 oder mindestens 2:1 oder mindestens 3:1 oder mindestens 4:1 oder bis zu 6:1 oder bis zu 5:1 oder bis zu 4,5:1 beträgt.

3. Amorphe, feste Dispersion nach Anspruch 1 oder 2, wobei die Brookfield-Viskosität bei 25 °C der linearen Poly(acrylsäure) mindestens 200 mPa·s (mindestens 200 cP) oder mindestens 250 mPa·s (mindestens 250 cP) oder mindestens 300 mPa·s (mindestens 300 cP) oder mindestens 400 mPa·s (mindestens 400 cP) beträgt, und/oder
nicht mehr als 3000 mPa·s (nicht mehr als 3000 cP) oder nicht mehr als 2500 mPa·s (nicht mehr als 2500 cP) oder nicht mehr als 2200 mPa·s (nicht mehr als 2200 cP) oder nicht mehr als 2100 mPa·s (nicht mehr als 2100 cP).

4. Amorphe, feste Dispersion nach einem der Ansprüche 1 bis 3, wobei die amorphe, feste Dispersion zu mindestens 15 Gew.-% lineare Poly(acrylsäure) oder zu mindestens 20 Gew.-% lineare Poly(acrylsäure) oder zu mindestens 25 Gew.-% lineare Poly(acrylsäure), und/oder
zu nicht mehr als 95 Gew.-% lineare Poly(acrylsäure) oder zu nicht mehr als 80 Gew.-% lineare Poly(acrylsäure) oder zu nicht mehr als 60 Gew.-% lineare Poly(acrylsäure) oder zu nicht mehr als 50 Gew.-% lineare Poly(acrylsäure) oder zu nicht mehr als 40 Gew.-% lineare Poly(acrylsäure) oder zu nicht mehr als 30 Gew.-% lineare Poly(acrylsäure) umfasst.

5. Amorphe, feste Dispersion nach einem der Ansprüche 1 bis 4, wobei die lineare Poly(acrylsäure) und der pharmazeutische Wirkstoff zusammen zu mindestens 90 Gew.-% oder zu mindestens 95 Gew.-% die amorphe, feste Dispersion umfassen.

6. Amorphe, feste Dispersion nach einem der Ansprüche 1 bis 5, wobei die amorphe, feste Dispersion zu nicht mehr als 10 Gew.-% Wasser oder zu nicht mehr als 5 Gew.-% Wasser oder zu nicht mehr als 1 Gew.-% Wasser oder kein Wasser umfasst.

7. Amorphe, feste Dispersion nach einem der Ansprüche 1 bis 6, wobei der pharmazeutische Wirkstoff in BCS-Klasse II oder BCS-Klasse IV ist.

8. Produkt, umfassend die amorphe, feste Dispersion nach einem der Ansprüche 1 bis 7 und gegebenenfalls
mindestens einen Hilfsstoff oder ein Adjuvans.

9. Produkt nach Anspruch 8, wobei das Produkt in einer Form vorliegt, die ausgewählt ist aus der Gruppe, die aus Granulaten, Kapseln, Pellets, Tabletten, Filmen und Implantaten besteht.

10. Verfahren zum Bilden einer amorphen, festen Dispersion eines pharmazeutischen Wirkstoffs, wobei das Verfahren umfasst:
Bilden einer flüssigen Dispersion, umfassend eine lineare Poly(acrylsäure), einen pharmazeutischen Wirkstoff und ein Lösungsmittelsystem, wobei die lineare Poly(acrylsäure) eine Brookfield-Viskosität bei 25 °C von mindestens 100 mPa·s (mindestens 100 cP) und ein gewichtsmittleres Molekulargewicht, bestimmt wie in der Beschreibung beschrieben, durch Größenausschlusschromatographie an einer flüssigen Probe von 1,5 g/l Polymer in 0,1 M NaNO₃ bei pH 10, von mindestens 200.000 g/mol (mindestens 200.000 Da) oder mindestens 250.000 g/mol (mindestens 250.000 Da) oder mindestens 300.000 g/mol (mindestens 300.000 Da) oder mindestens 400.000 g/mol (mindestens 400.000 Da) oder mindestens 500.000 g/mol (mindestens 500.000 Da) oder mindestens 600.000 g/mol (mindestens 600.000 Da) oder mindestens 800.000 g/mol (mindestens 800.000 Da) oder mindestens 1.000.000 g/mol (mindestens 1.000.000 Da) aufweist; und
Verdampfen des Lösungsmittelsystems aus der flüssigen Dispersion, um eine amorphe, feste Dispersion zu bilden,
wobei die amorphe, feste Dispersion zu mindestens 10 Gew.-% lineare Poly(acrylsäure) umfasst und wobei die lineare Poly(acrylsäure) und der pharmazeutische Wirkstoff zusammen zu mindestens 80 Gew.-% die amorphe, feste Dispersion umfassen.

11. Verfahren nach Anspruch 10, wobei ein Gewichtsverhältnis von pharmazeutischem Wirkstoff:linearer Poly(acrylsäure) in der flüssigen Dispersion mindestens 15:85 oder mindestens 30:70 oder mindestens 40:60 oder mindestens 50:50 oder mindestens 70:30 beträgt und/oder
wobei ein Gewichtsverhältnis von pharmazeutischem Wirkstoff:linearer Poly(acrylsäure) in der flüssigen Dispersion nicht mehr als 90:10 oder nicht mehr als 85:15 beträgt.

12. Verfahren nach einem der Ansprüche 10 bis 11, wobei die lineare Poly(acrylsäure) eine ist, die in einem Lösungsmittelsystem gebildet wurde, das im Wesentlichen wasserfrei ist.

13. Verfahren nach einem der Ansprüche 10 bis 12, wobei die lineare Poly(acrylsäure) eine ist, die in einem Lösungsmittelsystem gebildet wurde, das ausgewählt ist aus a) Ethylacetat und b) einer Mischung aus Ethylacetat und Cyclohexan.

14. Verfahren nach einem der Ansprüche 10 bis 13, wobei das Bilden der Dispersion der linearen Poly(acrylsäure) und des pharmazeutischen Wirkstoffs das Auflösen der linearen Poly(acrylsäure) in Pulverform in dem Lösungsmittelsystem oder in mindestens einem einer Vielzahl von in dem Lösungsmittelsystem verwendeten Lösungsmitteln umfasst, und/oder
wobei das Lösungsmittelsystem mindestens eines aus einem organischen, polaren, protischen Lösungsmittel und einem polaren, aprotischen Lösungsmittel umfasst, insbesondere wobei das Lösungsmittelsystem mindestens ein organisches, polares, protisches Lösungsmittel umfasst, das aus C₁-C₆-Alkoholen und Mischungen davon ausgewählt ist, und/oder wobei das Lösungsmittelsystem mindestens ein polares, aprotisches Lösungsmittel umfasst, das aus Dichlormethan, C₃-C₈-Ketonen, C₃-C₈-Ethern und Mischungen davon ausgewählt ist.

15. Verwendung der amorphen, festen Dispersion nach einem der Ansprüche 1 bis 7 oder des Produkts nach einem der Ansprüche 8 bis 9 oder der durch das Verfahren nach einem der Ansprüche 10 bis 14 gebildeten amorphen, festen Dispersion oder eines Produkts, das die durch das Verfahren nach einem der Ansprüche 10 bis 14 gebildete amorphe, feste Dispersion umfasst, zur Verabreichung eines pharmazeutischen Wirkstoffs.

## Revendications

1. Dispersion solide amorphe comprenant :
au moins 10 % en poids d'un poly(acide acrylique) linéaire ; et
un ingrédient actif pharmaceutique,
dans laquelle :
le poly(acide acrylique) linéaire a une viscosité Brookfield à 25 °C d'au moins 100 mPa·s (au moins 100 cP),
le poly(acide acrylique) linéaire et l'ingrédient pharmaceutique actif comprennent ensemble au moins 80 % en poids de la dispersion solide amorphe, et
le poly(acide acrylique) linéaire a une masse moléculaire moyenne en poids, déterminée comme décrit dans la description, par chromatographie d'exclusion stérique sur un échantillon liquide de 1,5 g/L de polymère dans 0,1 M NaNO₃ à pH 10, d'au moins 200 000 g/mol (au moins 200 000 Da) ou d'au moins 250 000 g/mol (au moins 250 000 Da), ou au moins 300 000 g/mol (au moins 300 000 Da), ou au moins 400 000 g/mol (au moins 400 000 Da), ou au moins 500 000 g/mol (au moins 500 000 Da), ou au moins 600 000 g/mol (au moins 600 000 Da), ou au moins 800 000 g/mol (au moins 800 000 Da), ou au moins 1 000 000 g/mol (au moins 1 000 000 Da).

2. Dispersion solide amorphe selon la revendication 1, dans laquelle un rapport en poids d'ingrédient pharmaceutique actif : le poly(acide acrylique) dans la dispersion solide amorphe est d'au moins 1:10, ou d'au moins 1:6, ou d'au moins 1:3, ou d'au moins 1:1,5, ou d'au moins 1:1, ou d'au moins 2:1, ou d'au moins 3:1, ou d'au moins 4:1, ou jusqu'à 6:1, ou jusqu'à 5:1, ou jusqu'à 4,5:1.

3. Dispersion solide amorphe selon la revendication 1 ou 2, dans laquelle la viscosité Brookfield à 25 °C du poly(acide acrylique) linéaire est d'au moins 200 mPa·s (au moins 200 cP), ou d'au moins 250 mPa·s (au moins 250 cP), ou d'au moins 300 mPa·s (au moins 300 cP), ou d'au moins 400 mPa·s (au moins 400 cP), et/ou
pas plus de 3 000 mPa·s (pas plus de 3 000 cP), ou pas plus de 2500 mPa·s (pas plus de 2 500 cP), ou pas plus de 2 200 mPa·s (pas plus de 2 200 cP), ou pas plus de 2 100 mPa·s (pas plus de 2 100 cP).

4. Dispersion solide amorphe selon l'une quelconque des revendications 1 à 3, dans laquelle la dispersion solide amorphe comprend au moins 15 % en poids de poly(acide acrylique) linéaire, ou au moins 20 % en poids de poly(acide acrylique) linéaire, ou au moins 25 % en poids de poly(acide acrylique) linéaire, et/ou
pas plus de 95 % en poids de poly(acide acrylique) linéaire, ou pas plus de 80 % en poids de poly(acide acrylique) linéaire, ou pas plus de 60 % en poids de poly(acide acrylique) linéaire, ou pas plus de 50 % en poids de poly(acide acrylique) linéaire, ou pas plus de 40 % en poids de linéaire poly(acide acrylique), ou pas plus de 30 % en poids de poly(acide acrylique) linéaire.

5. Dispersion solide amorphe selon l'une quelconque des revendications 1 à 4, dans laquelle le poly(acide acrylique) linéaire et l'ingrédient pharmaceutique actif représentent ensemble au moins 90 % en poids ou au moins 95 % en poids de la dispersion solide amorphe.

6. Dispersion solide amorphe selon l'une quelconque des revendications 1 à 5, dans laquelle la dispersion solide amorphe ne comprend pas plus de 10 % en poids d'eau, ou pas plus de 5 % en poids d'eau, ou pas plus de 1 % en poids d'eau, ou pas d'eau.

7. Dispersion solide amorphe selon l'une quelconque des revendications 1 à 6, dans laquelle l'ingrédient pharmaceutique actif est de classe II ou de classe IV.

8. Produit comprenant la dispersion solide amorphe selon l'une quelconque des revendications 1 à 7 et, éventuellement
au moins un excipient ou un adjuvant.

9. Produit selon la revendication 8, dans lequel le produit se présente sous une forme choisie dans le groupe constitué de granulés, capsules, pastilles, comprimés, films et implants.

10. Procédé de formation d'une dispersion solide amorphe d'un ingrédient actif pharmaceutique, le procédé comprenant :
la formation d'une dispersion liquide comprenant un poly(acide acrylique) linéaire, un ingrédient pharmaceutique actif et un système de solvants, le poly(acide acrylique) linéaire ayant une viscosité Brookfield à 25 °C d'au moins 100 mPa·s (au moins 100 cP) et une masse moléculaire moyenne en poids, déterminée comme décrit dans la description, par chromatographie d'exclusion stérique sur une un échantillon liquide de polymère 1,5 g/L dans 0,1 M NaNO₃ à pH 10, d'au moins 200 000 g/mol (au moins 200 000 Da), ou d'au moins 250 000 g/mol (au moins 250 000 Da), ou d'au moins 300 000 g/mol (au moins 300 000 Da), ou d'au moins 400 000 g/mol (au moins 400 000 Da), ou d'au moins 500 000 g/mol (au moins 500 000 Da), ou au moins 600 000 g/mol (au moins 600 000 Da), ou au moins 800 000 g/mol (au moins 800 000 Da), ou au moins 1 000 000 g/mol (au moins 1 000 000 Da) ; et
l'évaporation du système de solvants de la dispersion liquide pour former une dispersion solide amorphe,
dans lequel la dispersion solide amorphe comprend au moins 10 % en poids de poly(acide acrylique) linéaire et dans lequel le poly(acide acrylique) linéaire et l'ingrédient pharmaceutique actif représentent ensemble au moins 80 % en poids de la dispersion solide amorphe.

11. Procédé selon la revendication 10, dans lequel le rapport en poids entre l'ingrédient pharmaceutique actif et le poly(acide acrylique) linéaire dans la dispersion liquide est d'au moins 15:85, ou d'au moins 30:70, ou d'au moins 40:60, ou d'au moins 50:50, ou d'au moins 70:30 et/ou
dans lequel un rapport en poids de l'ingrédient pharmaceutique actif :poly(acide acrylique) linéaire dans la dispersion liquide n'est pas supérieur à 90:10, ou pas supérieur à 85:15.

12. Procédé selon l'une quelconque des revendications 10 à 11, dans lequel le poly(acide acrylique) linéaire est un poly(acide acrylique) qui a été formé dans un système de solvants qui est sensiblement exempt d'eau.

13. Procédé selon l'une quelconque des revendications 10 à 12, dans lequel le poly(acide acrylique) linéaire est celui qui a été formé dans un système de solvants choisi parmi a) acétate d'éthyle et b) un mélange d'acétate d'éthyle et de cyclohexane.

14. Procédé selon l'une quelconque des revendications 10 à 13, dans lequel la formation de la dispersion du poly(acide acrylique) linéaire et de l'ingrédient pharmaceutique actif comprend la dissolution du poly(acide acrylique) linéaire, sous forme de poudre dans le système de solvants ou dans au moins l'un parmi une pluralité de solvants utilisés dans le système de solvants, et/ou
dans lequel le système de solvants comprend au moins un solvant polaire protique organique et un solvant polaire aprotique, en particulier dans lequel le système de solvants comprend au moins un solvant polaire organique protique choisi parmi les alcools en C₁ à C₆, et des mélanges de ceux-ci, et/ou dans lequel le système de solvants comprend au moins un solvant polaire aprotique choisi parmi le dichlorométhane, les cétones en C₃-C₈, les éthers en C₃ à C₈, et des mélanges de ceux-ci.

15. Utilisation de la dispersion solide amorphe selon l'une quelconque des revendications 1 à 7 ou du produit selon l'une quelconque des revendications 8 à 9, ou de la dispersion solide amorphe formée par le procédé selon l'une quelconque des revendications 10 à 14, ou d'un produit comprenant la dispersion solide amorphe formée par le procédé selon l'une quelconque des revendications 10 à 14 pour l'administration d'un ingrédient actif pharmaceutique.
